# EUROPEAN PATENT APPLICATION

(11) **EP 3 412 297 A1**
(43) Date of publication of application: **12.12.2018**
(21) Application number: 17747705.6
(22) Date of filing: 01.02.2017
(51) Int. Cl.: A61K 35/15, A61K 35/12, A23L 33/10

(54) **COMPOSITION FOR INHIBITING CANCER METASTASIS USING APOPTOTIC CELLS**

(30) Priority: 01.02.2016 KR 20160012493; 01.12.2016 KR 20160162871
(71) Applicant: Ewha University-Industry Collaboration Foundation, Seoul 03760 (KR)
(72) Inventor: LEE, Jihee, Seoul 07976 (KR); KIM, Yong-Bae, Seoul 07535 (KR); AHN, Young-Ho, Seoul 07224 (KR)
(74) Representative: Office Freylinger
(86) International application number: PCT/KR2017/001049
(87) International publication number: WO 2017/135660

(57) **Abstract**

The present invention relates to a composition for inhibiting cancer metastasis using apoptotic cells. Particularly in this invention, macrophages and apoptotic cells were co-cultured to prepare a conditioned medium, which was treated to the mouse lung adenocarcinoma cell line 344SQ induced with EMT by using TGF-β1. As a result, EMT, migration and invasion of 344SQ were suppressed. In addition, EMT, migration and invasion of the human non-small cell lung cancer (NSCLS) cell line A549 were also inhibited by the treatment of the composition. Therefore, it was confirmed that the treatment of the composition of the invention could inhibit cancer metastasis and invasion in various human cancer cell lines originated from breast cancer, colon cancer and prostate cancer etc., equally to the above.

## Description

### BACKGROUND OF THE INVENTION

### 1. Field of the Invention

The present invention relates to a composition for inhibiting cancer metastasis which is conditioned medium (CM) from macrophages exposed to apoptotic cancer cells that has been confirmed to be effective in inhibiting cancer metastasis in the early stage, so that the composition of the present invention can be effectively used as a cancer metastasis inhibitor.

### 2. Description of the Related Art

Cancer is an abnormal cell mass generated by continuous division and proliferation of cells caused by destruction of the balance between cell division and death by any reason, which is also called tumor. In general, cancer is developed in more than 100 human body parts including organs, lymphocytes, bones and lymph nodes, and develops into serious symptoms through the phenomenon of invasion into surrounding tissues and metastasis to other organs (WHO, 2006).

EMT (epithelial-mesenchymal transition) is a kind of cell plasticity, which is precisely the cell differentiation from one type of cells to another type of cells, specifically from epithelial cells to mesenchymal cells (Zeisberg M, Neilson EG. Biomarkers for epithelial-mesenchymal transitions. J Clin Invest 2009; 119: 1429-1437). It was recently discovered that EMT is involved in different ways not only in tissue formation and differentiation in the fetal stage but also in tissue regeneration, fibrosis, cancer development and metastasis.

In the progress of cancer, local invasion and metastasis of cancer cells are observed, during which EMT (epithelial-mesenchymal transition) of epithelial tumor cells is observed. In this process, the transcriptional program of a specific gene is changed, the cell mobility increases, and the interaction between cell-cell or cell-extracellular matrix is changed to express hepatic phenotype.

In the course of cancer formation, cell adhesion materials such as E-cadherin in epithelial tumor cells are lost and thus epithelial tumor cells undergo morphological changes to be flat like fibroblasts. Epithelial-mesenchymal transition is known as one of the important early mechanisms of cancer development. When epithelial tumor cells are dedifferentiated, cell-cell and cell-basement bonds are lost, resulting in the obtainment of mobility. It is also known that epithelial-mesenchymal transition destroys basement membranes and initiates invasive growth and is involved in the process of metastasis of tumor cells to lymphatic vessels or blood vessels (Yoo, et al,. Kor J Oral Maxillofac Pathol 2009; 33(1): 37-46).

The biggest concern of life-threatening cancer is metastasis, and most of the deaths from cancer are explained by cancer metastasis. The clinically proven method of treating cancer is surgical operation. However, even after an original cancer is eliminated, a cancer patient is hard to be cured because of the migrated cancer cells. Therefore, cancer conquest can be regarded as a fight against cancer metastasis in practice. Although many studies have been conducted on the mechanism of cancer metastasis, they are not leading to the development of therapeutic drugs.

In the course of study to establish a method capable of effectively inhibiting cancer metastasis in the course of early cancer diagnosis, the present inventors confirmed that EMT, migration and invasion of cancer cells were efficiently suppressed in the conditioned medium (CM) from macrophages exposed to apoptotic cancer cells, leading to the completion of the present invention.

### SUMMARY OF THE INVENTION

It is an object of the present invention to provide a composition for inhibiting cancer metastasis using apoptotic cells.

To achieve the object above, the present invention provides a pharmaceutical composition comprising conditioned medium (CM) from macrophages exposed to apoptotic cancer cells for the inhibition of EMT (epithelial-mesenchymal transition), migration and invasion of cancer cells.

The present invention also provides a method for secreting an inhibitory material for EMT (epithelial-mesenchymal transition) of cancer cells from macrophages which comprises a step of co-culturing microphages and apoptotic cells.

The present invention also provides a preparation method of a composition for inhibiting cancer migration or invasion comprising a step of co-culturing microphages and apoptotic cells.

The present invention also provides a method for inhibiting EMT of cancer cells by treating conditioned medium (CM) from macrophages exposed to apoptotic cancer cells to cancer cells.

The present invention also provides a health functional food comprising conditioned medium (CM) from macrophages exposed to apoptotic cancer cells as an active ingredient for inhibiting cancer migration or invasion.

The present invention also provides an anticancer adjuvant comprising conditioned medium (CM) from macrophages exposed to apoptotic cancer cells as an active ingredient for inhibiting EMT (epithelial-mesenchymal transition), migration and invasion of cancer cells.

The present invention also provides a method for preventing or treating cancer comprising a step of administering conditioned medium (CM) from macrophages exposed to apoptotic cancer cells or apoptotic cancer cells to a subject with cancer.

The present invention also provides a method for inhibiting EMT (epithelial-mesenchymal transition), migration or invasion of cancer cells comprising a step of administering conditioned medium (CM) from macrophages exposed to apoptotic cancer cells or apoptotic cancer cells to a subject with cancer.

The present invention also provides a method for improving cancer treatment effect comprising a step of administering conditioned medium (CM) from macrophages exposed to apoptotic cancer cells or apoptotic cancer cells to a subject with cancer.

The present invention also provides a use of conditioned medium (CM) from macrophages exposed to apoptotic cancer cells or apoptotic cancer cells for the prevention or treatment of cancer.

The present invention also provides a use of conditioned medium (CM) from macrophages exposed to apoptotic cancer cells for inhibiting EMT (epithelial-mesenchymal transition), migration or invasion of cancer cells.

The present invention also provides a use of conditioned medium (CM) from macrophages exposed to apoptotic cancer cells for the preparation of a health functional food for inhibiting migration or invasion of cancer cells.

In addition, the present invention provides a use of conditioned medium (CM) from macrophages exposed to apoptotic cancer cells for the preparation of an anticancer adjuvant for inhibiting EMT (epithelial-mesenchymal transition), migration or invasion of cancer cells.

### ADVANTAGEOUS EFFECT

The present invention relates to a composition for inhibiting cancer metastasis using apoptotic cells. Particularly in this invention, macrophages and apoptotic cells were co-cultured to prepare a conditioned medium, which was treated to the mouse lung adenocarcinoma cell line 344SQ induced with EMT by using TGF-β1. As a result, EMT, migration and invasion of 344SQ were suppressed. In addition, EMT, migration and invasion of the human non-small cell lung cancer (NSCLS) cell line A549 were also inhibited by the treatment of the composition. Therefore, it was confirmed that the treatment of the composition of the invention could inhibit cancer metastasis and invasion in various human cancer cell lines originated from breast cancer, colon cancer and prostate cancer etc., equally to the above. The present inventors also confirmed the EMT inhibition effect in other conditioned media conditioned with various macrophages and additionally confirmed that such a mechanism was functioning Smad-independently. It was also observed that the PPARγ-dependent PTEN expression and secretion were increased in the macrophages stimulated by apoptotic cancer cells. It was confirmed that the secreted PTEN was involved in anti-EMT mechanism and cell polarity maintenance. It was also confirmed that the secretions of 15-lipoxygenase dependent 15-HETE and lipoxin A4 and PGD2 synthase dependent 15-deoxy-Δ12, 14-prostaglandin J2 (15d-PGJ2) were increased in the macrophages exposed on apoptotic cancer cells. The 15-lipoxygenase inhibitor PD146176 or lipocalin-type PGD synthase (L-PGDS) siRNA was injected in macrophages in order to inhibit the generation of PPARγ ligand. As a result, it was confirmed that the anti-EMT effect of the conditioned medium was reduced. In addition, when the PPARγ ligand was injected directly in epithelial cancer cells, EMT was inhibited, indicating that the secretion of PPARγ ligand induced by the interaction between apoptotic cancer cells and macrophages could inhibit EMT of epithelial cancer cells. In the mouse model, the expressions of PPARγ and PTEN were increased and the anti-metastasis effect was also confirmed. Therefore, the conditioned medium of the present invention or apoptotic cancer cells can be effectively used for a composition for inhibiting EMT, migration, stem cell transformation and invasion of cancer cells, or for cell therapy.

### BRIEF DESCRIPTION OF THE DRAWINGS

The application of the preferred embodiments of the present invention is best understood with reference to the accompanying drawings, wherein:

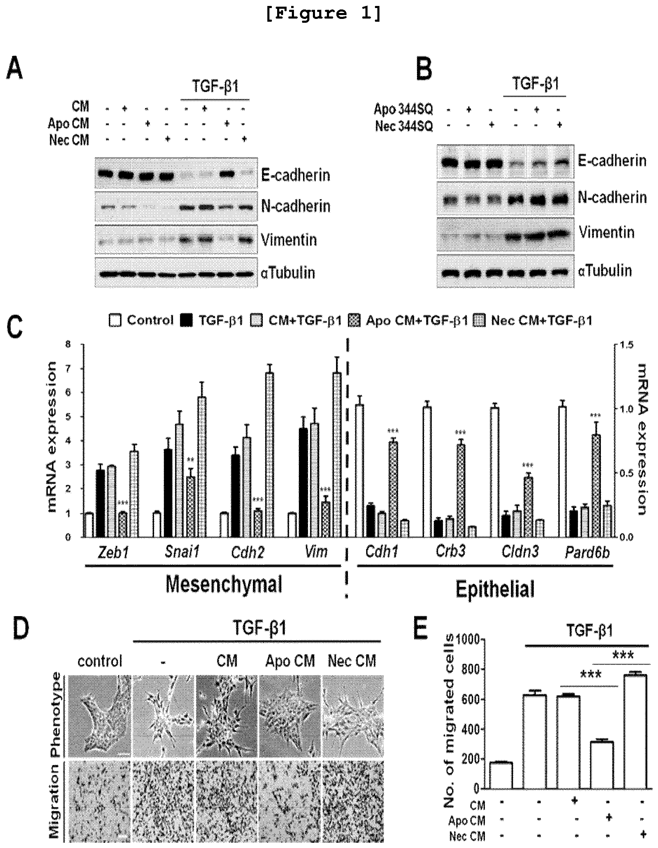

Figure 1 is a diagram illustrating that the TGF-β1 induced EMT and cancer cell migration were reduced in 344SQ cancer epithelial cells by the administration of the apoptotic conditioned medium (Apo CM) originated from the RAW 264.7 macrophages exposed on 344SQ apoptotic cells: In Figure 1a and in Figure 1c ∼Figure 1e, RAW 264.7 cells were stimulated by apoptotic (Apo) or necrotic (Nec) 344SQ epithelial cancer cells for 24 hours, resulting in the conditioned medium (CM). The prepared conditioned medium was treated to 344SQ epithelial cancer cells in the presence or absence of 10 ng/mℓ of TGF-β1 for 48 hours. In Figure 1b, 344SQ epithelial cancer cells were directly treated with apoptotic or necrotic 344SQ epithelial cancer cells in the presence or absence of 10 ng/mℓ of TGF-β1.
Figure 1a and Figure 1b present the results of immuno-blotting of total cell lysate using anti-E-cadherin, anti-N-cadherin and anti-vimentin antibodies.
Figure 1c presents the results of qPCR using Zeb1, Snail, Cdh2 and Vim as mesenchymal cell markers and Cdh1, Crb3, Cldn3 and Pard6a as epithelial cell markers under the conditions explained above. In the results, the control (white rod) is considered as the standard and each rod is presented as mean ± SEM (n=3). P value is determined by 2-tailed Student's test.
Figure 1d presents the visualization of cells using a phase contrast microscope of cells (top) to analyze migratory phenotypes and the evaluation thereof using fibronectin-coated transwells (bottom). All scale bars indicate 100 microns.
Figure 1e presents the quantification of the migrated cells visualized in Figure 1d (bottom) which is determined by counting the cells attached on the bottom of the top chamber. Each bar presents mean ± SEM by counting 3 sites from repeated wells.
Figure 2 is a diagram illustrating the inhibition of stem-likeness and invasiveness of 344SQ epithelial cancer cells by the apoptotic conditioned medium: RAW 264.7 cells were stimulated by apoptotic or necrotic 344SQ epithelial cancer cells for 24 hours. The conditioned medium was treated to 344SQ epithelial cancer cells for 48 hours in the presence or absence of 10 ng/mℓ of TGF-β1.
Figure 2a presents the results of qPCR using the stem cell markers CD90, Oct4, CD44 and CD133 under the conditions explained above. The control (white rod) is considered as the standard and each rod is presented as mean ± SEM (n=3). P value is determined by 2-tailed Student's test.
Figure 2b is a diagram illustrating the visualization of cells using a phase contrast microscope to analyze invasion capacity using matrigel coated transwells (left). The quantification of the invaded cells was determined by counting the cells attached on the bottom of the top chamber (right). Each bar presents mean ± SEM by counting 3 sites from repeated wells.
Figure 2c is a diagram illustrating the inhibition of the disruption of cell polarity by the apoptotic conditioned medium. 344SQ epithelial cancer cells were cultured in 3D matrigel with the same condition as described in the example, as shown in the picture. All scale bars indicate 100 microns.
Figure 3 is a diagram illustrating the inhibition of EMT and migration of cancer cells by the apoptotic conditioned medium in non-small cell lung cancer: In Figure 3a and Figure 3c ∼ Figure 3e, RAW 264.7 microphages were stimulated by apoptotic or necrotic A549 epithelial cancer cells for 24 hours. The conditioned medium was treated to A549 epithelial cancer cells for 48 hours in the presence or absence of TGF-β1. In Figure 3b, apoptotic or necrotic 344SQ epithelial cancer cells were treated directly to 344SQ epithelial cancer cells in the presence or absence of 10 ng/mℓ of TGF-β1.
Figure 3a and Figure 3b present the results of immuno-blotting of total cell lysate using anti-E-cadherin, anti-N-cadherin and anti-vimentin antibodies.
Figure 3c presents the results of qPCR using Snail, Cdh2 and Vim as mesenchymal cell markers and Cdh1, Pard6b and OCLN as epithelial cell markers under the conditions explained above. In the results, the control (white rod) is considered as the standard and each rod is presented as mean ± SEM (n=3). P value is determined by 2-tailed Student's test.
Figure 3d presents the visualization of cells using a phase contrast microscope of cells (top) to analyze migratory phenotypes and the evaluation thereof using fibronectin-coated transwells (bottom). All scale bars indicate 100 microns.
Figure 3e presents the quantification of the migrated cells (Figure d, bottom) which is determined by counting the cells attached on the bottom of the top chamber. Each bar presents mean ± SEM by counting 3 sites from repeated wells.
Figure 4 is a diagram illustrating the inhibition of cancer cell stemness and invasiveness by the apoptotic conditioned medium: RAW 264.7 cells were stimulated by apoptotic or necrotic A549 epithelial cancer cells for 24 hours. The conditioned medium was treated to A549 epithelial cancer cells for 48 hours in the presence or absence of 10 ng/mℓ of TGF-β1.
Figure 4a presents the results of qPCR using the stem cell markers ALDH1A, CD44 and CD90 under the conditions explained above. The control (white rod) is considered as the standard and each rod is presented as mean ± SEM (n=3). P value is determined by 2-tailed Student's test.
Figure 4b is a diagram illustrating the visualization of cells using a phase contrast microscope to analyze invasion capacity using matrigel coated transwells (left). The quantification of the invaded cells was determined by counting the cells attached on the bottom of the top chamber (right). Each bar presents mean ± SEM by counting 3 sites from repeated wells. All scale bars indicate 100 microns.

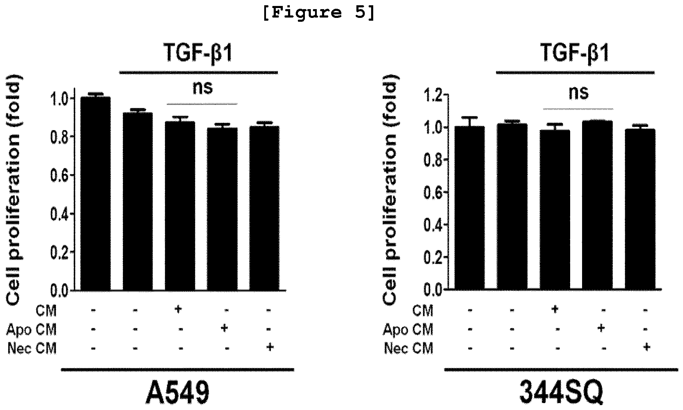

Figure 5 is a diagram illustrating that the conditioned medium did not affect the cellular proliferative activity in lung cancer cells: Lung cancer cells (344SQ and A549, 1 × 10⁴ cells/well) were distributed in a 96-well culture plate by 3 sets, followed by culture in RPMI 1640 supplemented with 10% FBS for overnight. Then, the culture medium was replaced with the conditioned medium of the invention, followed by culture for 48 hours. MTT assay was performed at 37°C for 4 hours. Cells were lysed in dimethyl sulfoxide (100 *µ*ℓ/well), followed by culture in an orbital shaker for 30 minutes in the darkness at room temperature. Cell proliferation was measured at 570 nm absorbance using a microplate reader.

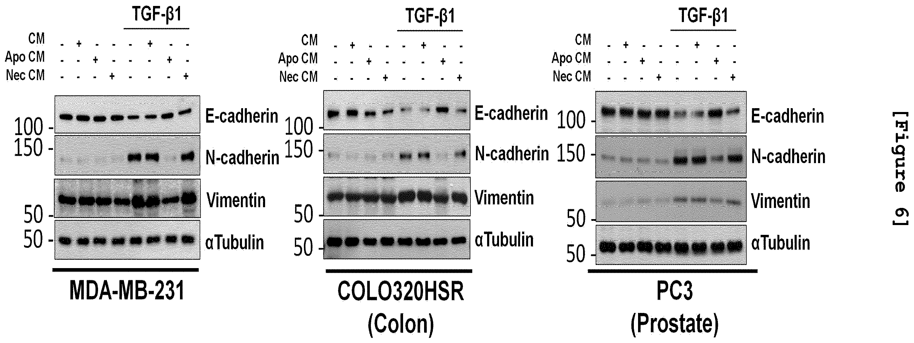

Figure 6 is a diagram illustrating the anti-EMT effect of the apoptotic conditioned medium in various human cancer cell lines treated with TGF-β1: Immuno-blotting was performed with various cancer cells treated with the conditioned media obtained from RAW264.7/apoptotic cancer cells (MDA-MB-231, COLO320HSR or PC3; Apo CM) or RAW 264.7/necrotic cancer cells (Nec CM) for 48 hours in the presence or absence of TGF-β1 (10 ng/mℓ). Immuno-blotting was performed with the total cell lysate using EMT markers (E-cadherin, N-cadherin and vimentin) and α-tubulin antibody. Each panel indicates the anti-EMT effect of the apoptotic conditioned medium in such cell lines as MDA-MB-23 (breast cancer, left), COLO320HSR (colon cancer, middle) and PC3 (prostate cancer, right).
Figure 7 is a diagram confirming that TGF-β1 induced EMT was inhibited in 344SQ epithelial cancer cells exposed on the apoptotic conditioned medium originated from mouse BMDMs (bone marrow-derived macrophages) or M2-like BMDMs: In Figure 7a and Figure 7b, mouse BMDMs (bone marrow-derived macrophages) or M2-like BMDMs were stimulated by apoptotic 344SQ epithelial cancer cells for 24 hours. Then, the conditioned medium was treated to 344SQ epithelial cancer cells for 48 hours in the presence of absence of 10 ng/mℓ of TGF-β1.
Figure 7a and Figure 7b present the results of immuno-blotting of total cell lysate using anti-E-cadherin, anti-N-cadherin and anti-vimentin antibodies.

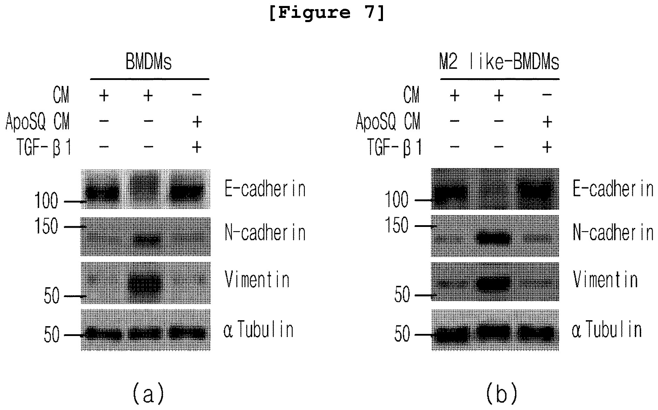

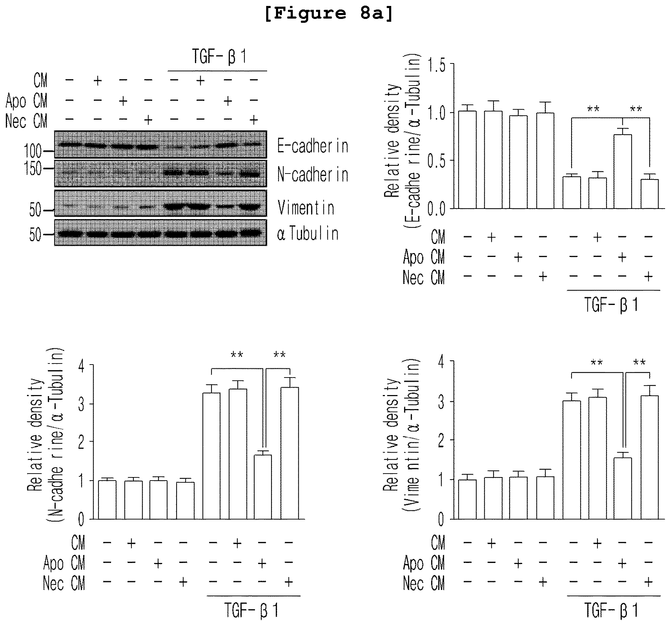

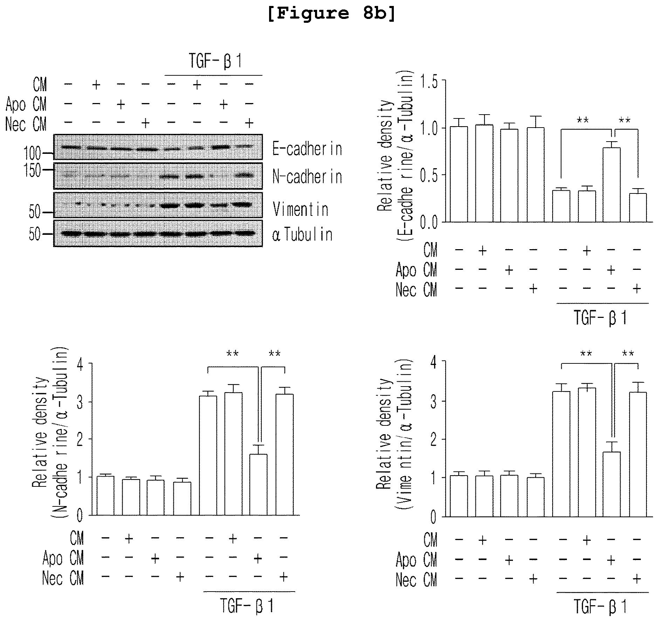

Figure 8 is a diagram illustrating the inhibition of EMT induced by TGF-β1 in A549 epithelial cancer cells by the treatment of the conditioned medium obtained from human blood monocyte-derived macrophages (MDMs) of healthy donors or MDMs of lung cancer patients stimulated by apoptotic or necrotic A549 epithelial cancer cells: In Figure 8a and Figure 8b, A549 epithelial cancer cells were treated with the conditioned medium obtained from human blood monocyte-derived macrophages of healthy donors (Figure 8a) or human blood monocyte-derived macrophages of lung cancer patients (Figure 8b) stimulated by apoptotic or necrotic A549 epithelial cancer cells in the presence or absence of 10 ng/mℓ of TGF-β1 according to the same conditions as described in Figure 7.
Figure 8a and Figure 8b present the results of immuno-blotting of total cell lysate using anti-E-cadherin, anti-N-cadherin and anti-vimentin antibodies.
Figure 9 is a set of diagrams confirming that the EMT inhibition effect of the apoptotic conditioned medium was Smad signal independent:
   Figure 9a presents the results of immune-blotting. Particularly, 344SQ epithelial cancer cells were treated with 10 ng/mℓ of TGF-β1 over different times and then immune-blotting was performed to investigate Smad2, Smad3, ERK1/2, p38 and AKT which are the signal transducing proteins known to be activated by TGF-β1.
   Figures 9b, 9c, 9d and 9f present the results of immune-blotting, wherein 10 ng/mℓ of TGF-β1 was added to the conditioned medium obtained from RAW264.7 cells stimulated by apoptotic 344SQ epithelial cancer cells. The prepared conditioned medium was treated to 344SQ epithelial cancer cells. Then, the signal transducing proteins such as Smad2, Smad3, ERK1/2, p38 and AKT were analyzed by immune-blotting.
Figure 10 is a set of diagrams illustrating the expressions of PPARγ and PTEN mRNAs and proteins were increased in macrophages stimulated by apoptotic cancer cells:
   Figures 10a, 10b, 10c, 10d and 10e present the results of qPCR and immune-blotting performed to investigate the expressions of PPARγ and PTEN mRNAs and proteins in macrophages stimulated by apoptotic 344SQ cancer cells (ApoSQ).
   Figure 10f presents the results of qPCR investigating the PTEN mRNA expression in blood monocyte-derived macrophages (MDMs) of healthy donors or MDMs of lung cancer patients stimulated by apoptotic A549 epithelial cancer cells (ApoA).
Figure 11 is a set of diagrams illustrating that the up-regulation of PPARγ and PTEN mRNAs and proteins in macrophages stimulated by apoptotic 344SQ epithelial cancer cells (ApoSQ) was suppressed by the PPARγ inhibitor GW9662 or PPARγ siRNA:
   Figure 11a and Figure 11b present the results of qPCR and immune-blotting to investigate the expressions of PPARγ and PTEN mRNAs and proteins in RAW264.7 cells stimulated by apoptotic 344SQ epithelial cancer cells after the treatment of GW9662. Figure 11c presents the results of measuring PPARγ activity using nuclear extracts.
   Figure 11d presents the results of immune-blotting performed to analyze the expressions of PPARγ and PTEN proteins which had been increased by the introduction of PPARγ siRNA in RAW264.7 cells stimulated by apoptotic 344SQ epithelial cancer cells.
   Figure 11e and Figure 11f present the results of immune-blotting performed to investigate the expression patterns of PPARγ and PTEN proteins in BMDMs (mouse bone marrow-derived macrophages) and M2-like BMDMs stimulated by apoptotic 344SQ epithelial cancer cells according to the treatment of GW9662.
Figure 12 is a set of diagrams illustrating the inhibition of the anti-EMT activity of the apoptotic conditioned medium in RAW 264.7 cells stimulated by apoptotic 344SQ cancer cells (ApoSQ) by the treatment of the PPARγ inhibitor GW9662:
   Figure 12a illustrates the changes of cell morphology observed with a phase contrast microscope to investigate the anti-EMT inhibitory effect.
   Figure 12b presents the results of immuno-blotting of total cell lysate using anti-E-cadherin, anti-N-cadherin and anti-vimentin antibodies performed to investigate the anti-EMT inhibitory effect.
   Figure 12c presents the results of qPCR performed to investigate the expression patterns of mRNAs of the transcription factors *Snai1* and *Zeb1.*
   Figure 12d is a diagram illustrating the visualization of cells using a phase contrast microscope to analyze invasion capacity using matrigel coated transwells (left). The quantification of the invaded cells was determined by counting the cells attached on the bottom of the top chamber (right). Each bar presents mean ± SEM by counting 3 sites from repeated wells. All scale bars indicate 100 microns.
Figure 13 is a set of diagrams illustrating the PPARγ-dependent PTEN secretion in macrophages stimulated by apoptotic cancer cells:
   Figure 13a presents the exosome secretion state of RAW 264.7 cells stimulated by apoptotic 344SQ epithelial cancer cells observed by transmission electron microscope (TEM).
   Figure 13b presents the results of immune-blotting performed to investigate the changes of PTEN expression and secretion using immunoprecipitation with the pulverized BMDM cells and the conditioned medium wherein the PTEN expression was induced by apoptotic 344SQ epithelial cancer cells.
   Figure 13c presents the results of immune-blotting and immunoprecipitation using PTEN antibody and the exosome marker CD63 antibody after separating exosome by step-wise ultracentrifugation.
Figure 14 is a set of diagrams illustrating the AKT phosphorylation and the PTEN expression pattern in 344SQ epithelial cancer cells introduced with PTEN:
Figure 14a presents the results of immune-blotting performed to compare the PTEN expression pattern and the AKT phosphorylation over the time according to the treatment of the apoptotic conditioned medium.
Figure 14b and Figure 14c present the results of immune-blotting to investigate the PTEN protein expression according to the dilution ratio of the apoptotic conditioned medium and qPCR performed to investigate the time-dependent PTEN mRNA expression.
Figure 14d presents the results of immune-blotting performed to investigate the effect of TGF-β1 itself on the PTEN protein expression.
Figure 15 is a set of diagrams illustrating the introduced PTEN dependent AKT and p38 MAP kinase phosphorylation inhibition according to the administration of the apoptotic conditioned medium to 344SQ epithelial cancer cells:
   Figure 15a presents the result of immune-blotting performed to compare the PTEN protein expression and the AKT phosphorylation over the time after the treatment of the apoptotic conditioned medium with 10 ng/mℓ of TGF-β1.
   Figures 15b, 15c and 15d present the results of immune-blotting performed to investigate whether or not the TGF-β1-induced AKT and p38 phosphorylation inhibition could be recovered in 344SQ epithelial cancer cells treated with the conditioned medium obtained from RAW264.7 cells introduced with PTEN siRNA.
Figure 16 is a set of diagrams illustrating the inhibition of cell polarity decrease in 344SQ epithelial cancer cells introduced with PTEN:
   Figure 16a presents the results of immune-blotting performed to investigate the PTEN protein expression pattern and the AKT phosphorylation pattern in cell membrane containing Na⁺/K⁺ ATPase richly after treating 344SQ epithelial cancer cells with the apoptotic conditioned medium.
   Figure 16b presents the results of observing cells under a confocal microscope to confirm the disruption of cell polarity induced by TGF-β1 in 344SQ epithelial cancer cells treated with the apoptotic conditioned medium obtained from RAW 264.7 cells introduced with control siRNA or PTEN siRNA.
   Figure 16c presents the results of observing cells under a confocal microscope to confirm the disruption of intercellular contact induced by TGF-β1 in the cells treated with the apoptotic conditioned medium obtained from RAW 264.7 cells introduced with control siRNA or PTEN siRNA.
   Figure 16d and Figure 16e present the results of observing cell polarity in the cells cultured in 3D matrigel and the results of observing cells under a phase contrast microscope performed to investigate the disruption of cell polarity in the cells cultured in 3D matrigel and cells under a confocal microscope performed to investigate the intercellular contact according to the treatment of SF1670.
Figure 17 is a set of diagrams confirming the anti-EMT and the anti-invasion effect of the apoptotic conditioned medium in 344SQ epithelial cancer cells was PTEN dependent:
   Figure 17a presents the result of immune-blotting of total cell lysate using anti-E-cadherin, anti-N-cadherin and anti-vimentin antibodies, performed to investigate the anti-EMT effect in 344SQ epithelial cancer cells treated with the apoptotic conditioned medium obtained from RAW 264.7 cells introduced with control siRNA or PTEN siRNA.
   Figure 17b and Figure 17c present the results of the changes of the expressions of mRNAs of the transcription factors Snail and Zeb1 the changes of the expressions of mRNAs of the transcription factors Snail and Zeb1 using qPCR and penetration ability analysis using matrigel coated transwells performed to investigate invaded cell numbers.
Figure 18 is a set of diagrams illustrating the anti-EMT inhibitory effect by the PTEN activation in 344SQ epithelial cancer cells treated with the apoptotic conditioned medium:
   Figures 18a, 18b and 18c present the results of the phase contrast microscope observation, immune-blotting, qPCR and transwell assay performed to investigate the inhibition of the anti-EMT and the anti-invasion activity according to the treatment of the apoptotic conditioned medium along with TGF-β1 after pre-treating 344SQ epithelial cancer cells with the PTEN selective inhibitor SF1670.
Figure 19 is a set of diagrams illustrating the generation of PPARγ ligands in macrophages stimulated by apoptotic cancer cells:
   Figures 19a, 19b and 19c presents the difference of the concentrations of 15(S)-HETE, lipoxin A4 and 15d-PGJ₂ in the apoptotic conditioned medium and the necrotic conditioned medium.
   Figures 19d, 19e, 19f and 19g present the changes of the content of PPARγ ligands according to the treatment of the conditioned medium. Particularly, in order to investigate whether or not the secreted PPARγ ligands could be involved in the increase of PPARγ activity, the 15-lipoxygenase selective inhibitor PD146176 and lipocalin-type PGDS siRNA were used for the experiment.
Figure 20 is a set of diagrams illustrating that the effect of the apoptotic conditioned medium to reverse the inhibition of the TGF-β1-induced PPARγ mRNA expression, activity and PTEN mRNA and protein expression when the generation of PPARγ ligand secreted in macrophages stimulated by apoptotic cancer cells was suppressed:
   Figures 20a, 20b, 20c, 20d, 20e, 20f, 20g and 20h present the results of qPCR, PPARγ activity measurement and immune-blotting performed to investigate the inhibition of PPARγ mRNA activity induced by TGF-β1 in the culture medium wherein PPARγ ligand was down-regulated which was generated in macrophages introduced with PD146176 or siL-PGDS and to investigate whether or not the PTEN mRNA and protein inhibition effect therein could be reversed.
Figure 21 is a set of diagrams illustrating that the PPARγ ligand secreted in macrophages stimulated by apoptotic cancer cells was involved in the anti-EMT mechanism by the apoptotic conditioned medium:
   In Figures 21a, 21b, 21c and 21d, qPCR was performed to analyze the *Snai1* and *Zeb1* mRNA expression patterns in order to investigate whether or not the anti-EMT activity was still working in the culture medium with reduced PPARγ caused by the introduction of PD146176 or siL-PGDS in macrophages. Immuno-blotting of total cell lysate was also performed using anti-E-cadherin, anti-N-cadherin and anti-vimentin antibodies to investigate the expression patterns of the EMT marker proteins.
Figure 22 is a set of diagrams illustrating the anti-EMT effect in cancer cells treated with PPARγ ligand:
   Figure 22a and Figure 22b present the results of immune-blotting and qPCR performed to investigate the TGF-β1-induced EMT inhibition effect of PPARγ ligand at the basic concentration and the stimulation concentration.
   Figures 22c, 22d, 22e and 22f present the results of qPCR, PPARγ activity measurement and immune-blotting performed to investigate the inhibition of TGF-β1-induced reduction of PPARγ mRNA activity by PPARγ ligand at the basic concentration and to investigate whether or not the PTEN mRNA and protein inhibition effect could be reversed.
Figure 23 is a set of diagrams illustrating the *in vivo* anti-metastasis effect of apoptotic cancer cells in the mouse tumor:
   In Figures 23a, 23b, 23c, 23d and 23e, 344SQ epithelial cancer cells were transplanted in a mouse model. 2 days later, apoptotic 344SQ epithelial cancer cells were injected in the mouse. 6 weeks later, the formation and the weight of a primary tumor and the metastasis thereof to the lung were investigated and the results are shown in those Figures.
Figure 24 is a set of diagrams illustrating the up-regulation of the anti-metastasis related molecules in the mouse primary tumor by apoptotic cancer cells:
   Figures 24a, 24b, 24c, 24d and 24e present the results of qPCR performed to investigate the expression patterns of PPARγ, PTEN, CD36, *Snai1* and *Zeb1* mRNAs in the mouse primary tumor after injection with apoptotic 344SQ epithelial cancer cells.
   Figure 24f presents the results of immune-blotting performed to investigate the changes of the PTEN protein expression and the AKT phosphorylation.
Figure 25 is a set of diagrams illustrating the results of immunohisto staining confirming the up-regulation of PPARγ in the mouse primary tumor and tumor-infiltrating macrophages after the treatment of apoptotic cancer cells:
   Figures 25a, 25b, 25c and 25d present the results of immunohisto staining performed to investigate the PPARγ and F4/80 expression patterns.
Figure 26 is a set of diagrams illustrating the results of immunohisto staining confirming the up-regulations of PTEN and CD36 in the mouse primary tumor and tumor-infiltrating macrophages after the treatment of apoptotic cancer cells:
   Figures 26a, 26b, 26c, 26d, 26e and 26f present the results of immunohisto staining performed to investigate the PTEN, CD36 and F4/80 expression patterns.

### DESCRIPTION OF THE PREFERRED EMBODIMENTS

Hereinafter, the present invention is described in detail.

The present invention provides a pharmaceutical composition comprising conditioned medium (CM) from macrophages exposed to apoptotic cancer cells for the inhibition of EMT (epithelial-mesenchymal transition), migration and invasion of cancer cells.

The macrophage above can be originated from a mouse or human, which is preferably selected from the group consisting of microglia, histocytes, Hofbauer cells, mesangial cells, Kupffer cells, peritoneal macrophages, alveolar macrophage, epidermal or dermal macrophages, marginal zone macrophages, metallophilic macrophages, red pulp macrophages, white pulp macrophages and osteoclasts.

The apoptotic cell above is preferably epithelial cancer cell. In a preferred embodiment of the present invention, it is preferably epithelial cancer cell.

The apoptotic cell above is preferably prepared by irradiating epithelial cancer cells with UV at the wavelength of 100 ∼ 400 nm for 5 ∼ 30 minutes, more preferably at the wavelength of 150 ∼ 350 nm for 5 ∼ 20 minutes and most preferably at the wavelength of 200 ∼ 300 nm for 10 ∼ 15 minutes, but not always limited thereto.

After irradiating the epithelial cancer cells above, the cells can be cultured in RPMI 1604 medium supplemented with 10% FBS in 1 ∼ 10% CO₂ at 30 ∼ 40 °C for 1 ∼ 5 hours.

The culture herein is preferably performed in X-VIVO or serum free DMEM for 20 ∼ 30 hours before the macrophages are stimulated by apoptotic cells. In a preferred embodiment of the present invention, it is more preferred that the cells are cultured in X-VIVO medium for 24 hours to make them serum-deficient. To stimulate the apoptotic cells, the culture medium can be replaced with X-VIVO or serum-free DMEM containing apoptotic cells. The culture preferably continued for 10 ∼ 30 more hours after the medium replacement, and more preferably continued for 15 ∼ 25 hours and most preferably for 18 ∼ 24 hours.

The culture fluid above characteristically inhibits EMT (epithelial-mesenchymal transition) which is known as the pre-stage of cancer metastasis. So, the culture fluid above is characterized by suppressing the primary stage of cancer metastasis, which is cancer cell migration and invasion, by inhibiting EMT. EMT is a phenomenon in which epithelial cells are changed into mesenchymal cells. In the process of cancer formation and metastasis, such a cell adhesion material as E-cadherin is lost and a morphological change is induced, so that a change in epithelial tumor cells is induced, by which EMT plays various roles in the progress and metastasis of cancer. In a preferred embodiment of the present invention, EMT was induced by treating cells with TGF-β1.

The cancer herein is preferably selected from the group consisting of lung cancer, breast cancer, colon cancer and prostate cancer and the lung cancer herein is preferably non-small cell lung cancer, but not always limited thereto.

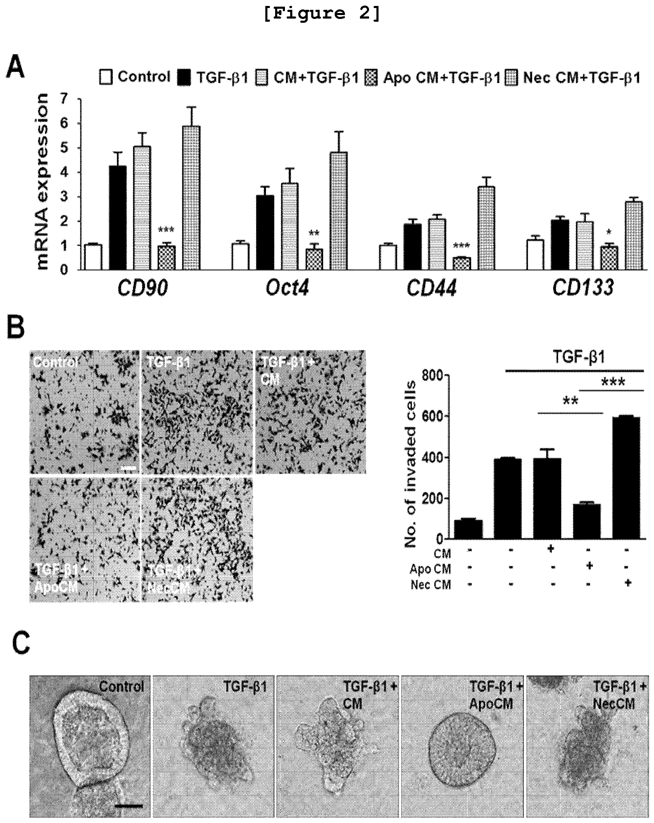

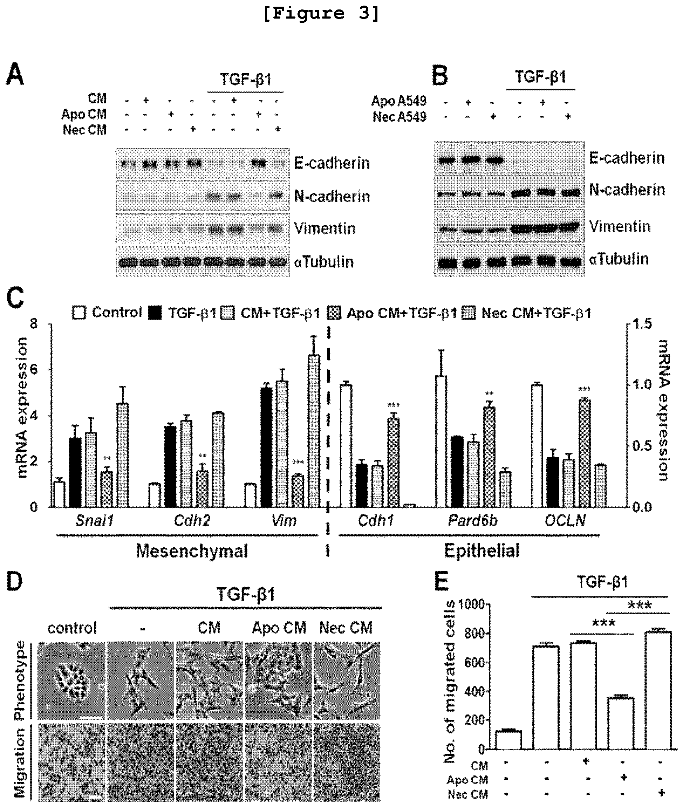

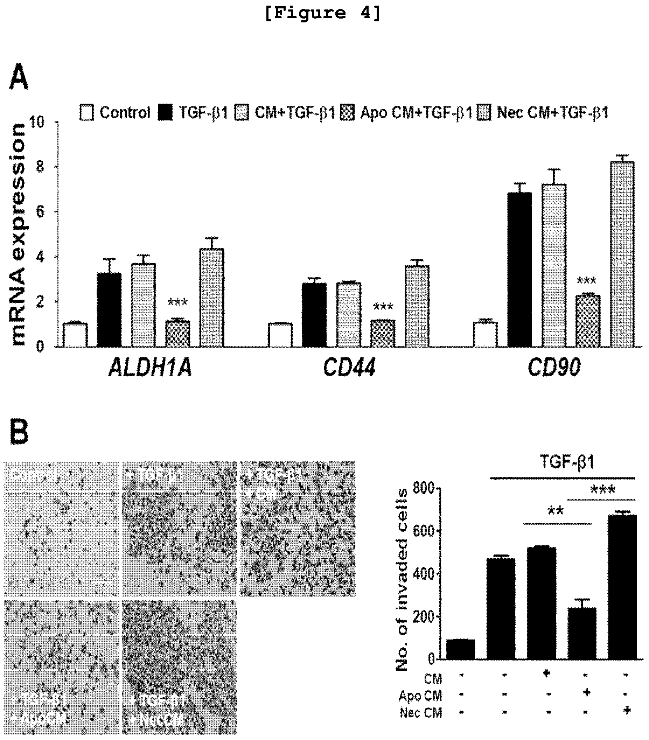

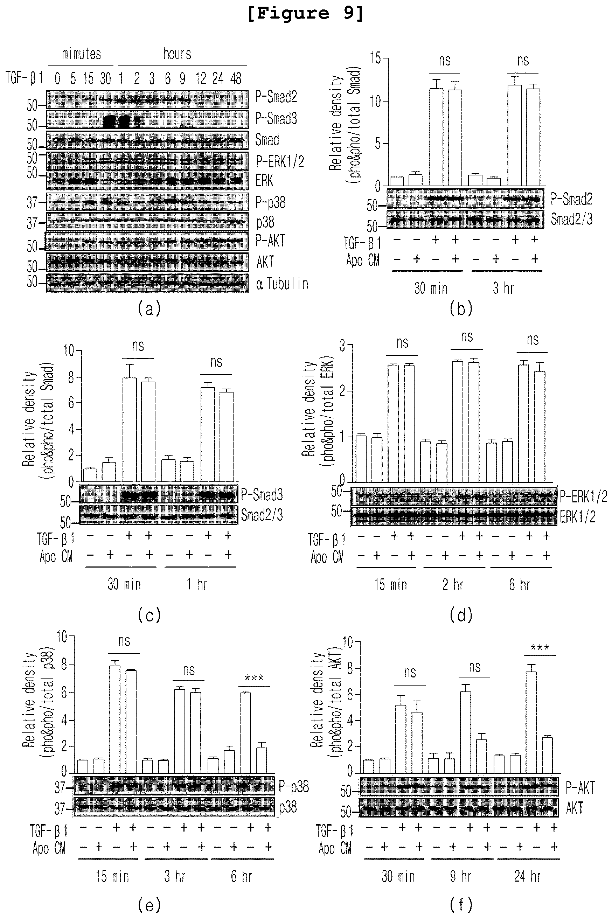

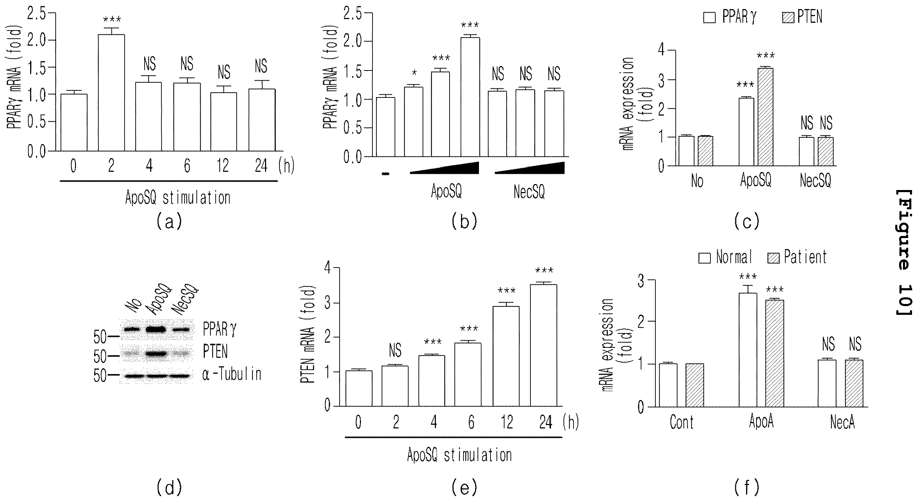

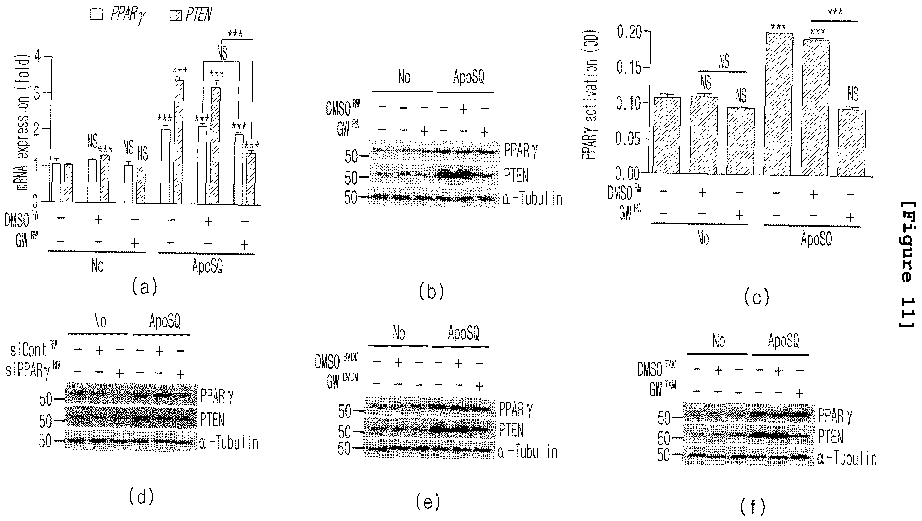

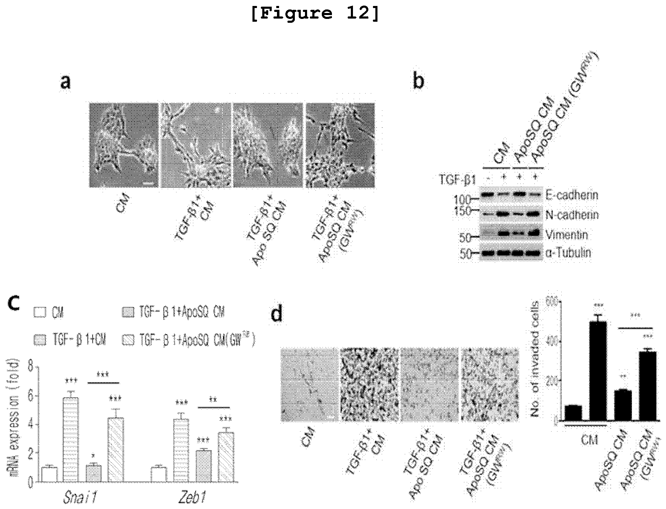

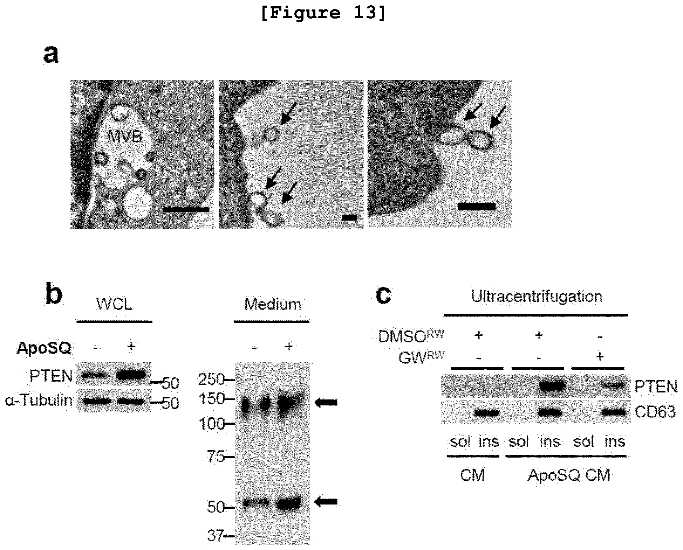

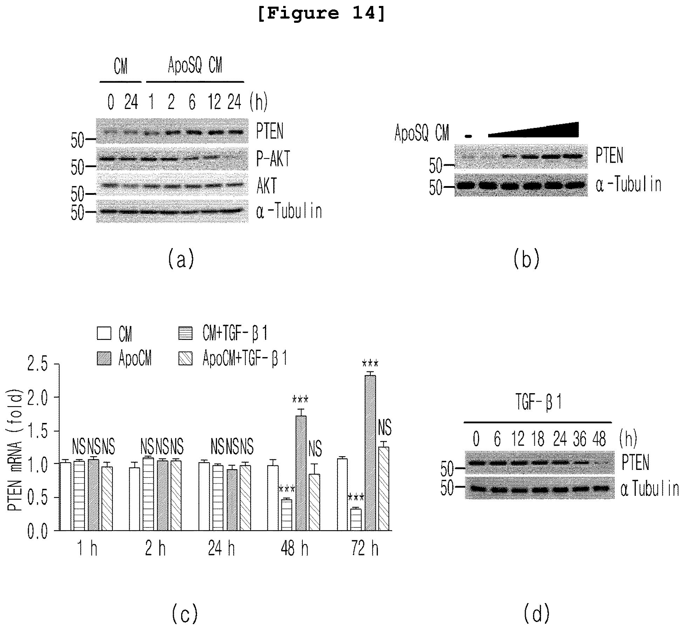

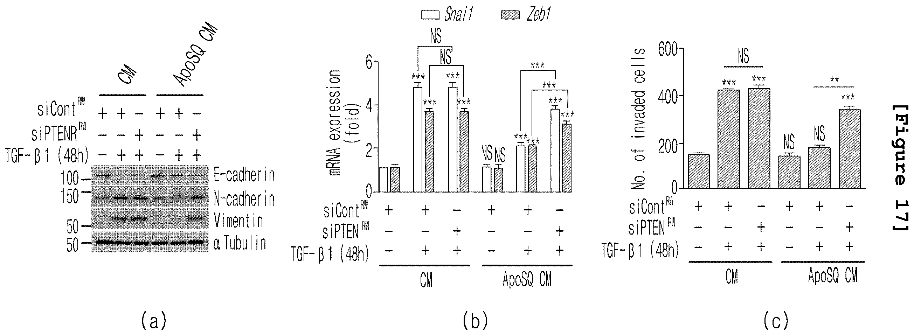

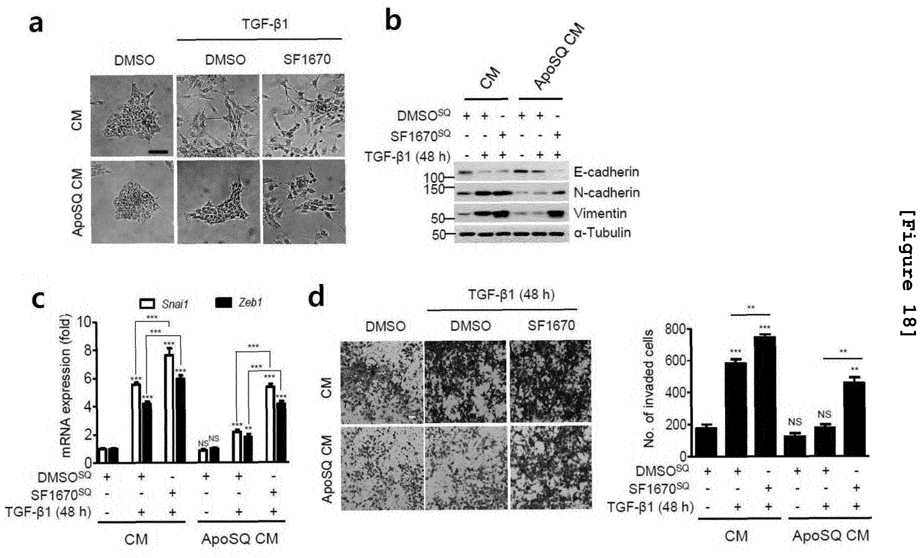

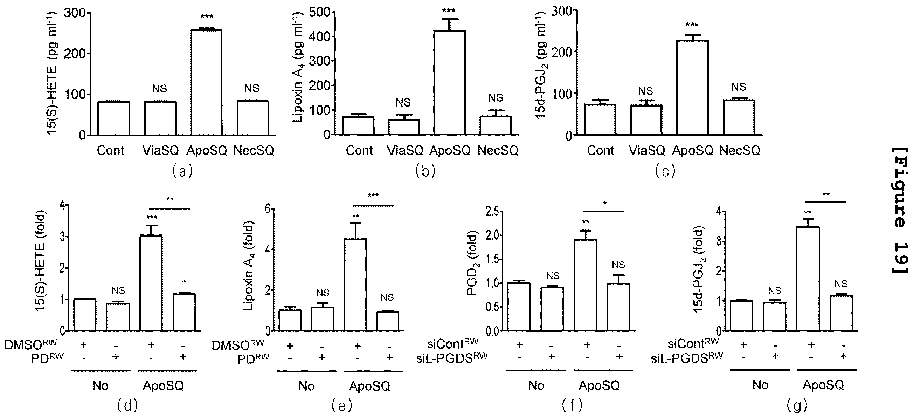

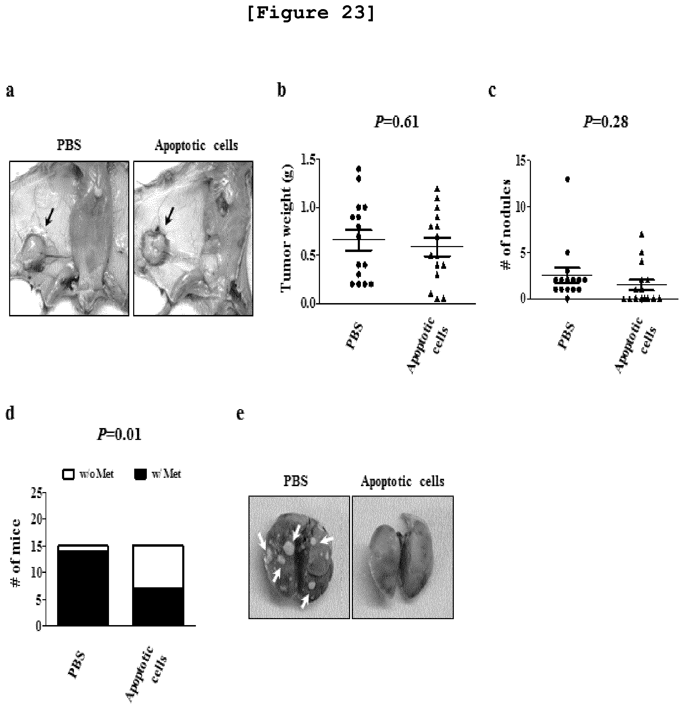

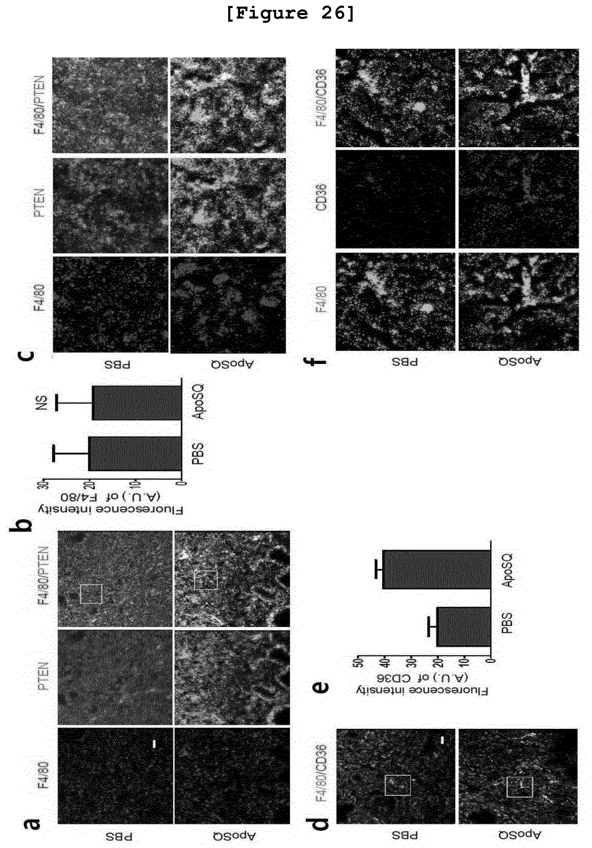

In a preferred embodiment of the present invention, the present inventors prepared the conditioned medium by co-culturing macrophages and apoptotic cancer cells together. The prepared conditioned medium was treated to the mouse lung adenocarcinoma cell line 344SQ wherein EMT was induced by TGF-β1. As a result, the present inventors confirmed that EMT (epithelial-mesenchymal transition), migration, metastasis and invasion of cancer cells were all inhibited by investigating protein expression and mRNA expression, by observing phenotype using a phase contrast microscope and by performing fibronectin treated transwell assay (see Figures 1 and 2). The inhibition of EMT, migration and invasion of cancer cells in human NSCLS was also confirmed (see Figures 3 and 4). The effect above did not affect on the cell proliferation activity (see Figure 5). The present inventors further confirmed the inhibition of EMT, migration and invasion of cancer cells in various human cancer cell lines originated from breast cancer, colon cancer and prostate cancer equally to the above (see Figure 6). The present inventors also confirmed the EMT inhibition effect in various macrophage-conditioned media (see Figures 7 and 8). The mechanism above worked Smad independently (see Figure 9). The up-regulation of PPARγ and PTEM mRNA and protein was confirmed in macrophages stimulated by apoptotic cancer cells (see Figure 10). It was also confirmed that the expressions of PPARγ and PTEN mRNAs were inhibited by a PPARγ inhibitor (see Figure 11). The secreted PTEN macrophages stimulated by apoptotic cancer cells was involved in the anti-EMT mechanism and PTEN was secreted PPARγ-dependently in the macrophages above (see Figures 12 and 13). It was confirmed that the cell signaling mechanism and cell polarity disruption were inhibited in 344SQ epithelial cancer cells introduced with PTEN (see Figures 14 ∼ 16). The anti-EMT inhibitory effect was also observed in the cells (see Figures 17 and 18). The PPARγ ligand secreted in macrophages stimulated by apoptotic cancer cells and the treated PPARγ ligand were also confirmed to be involved in the anti-EMT mechanism (see Figures 19 ∼ 22). After confirming the *in vivo* anti-metastasis effect of apoptotic cancer cells in a real mouse tumor, the inventors further confirmed the up-regulation of PPARγ and PTEN in the tumor (see Figures 23 and 26).

Therefore, the apoptotic conditioned medium of the present invention can be effectively used for inhibiting EMT, migration and invasion of cancer cells and further for a composition for inhibiting cancer metastasis due to the inhibition effect above.

The pharmaceutical composition of the present invention can additionally include generally used excipients, disintegrants, sweeteners, lubricants, flavors and the like and can be formulated as tablets, capsules, powders, granules, suspensions, emulsions, syrups, and other liquid preparations.

Particularly, the pharmaceutical compositions of the present invention can be formulated for oral administration, for example, tablets, troches, lozenges, aqueous or oily suspensions, powders or granules, emulsions, hard or soft capsules, and syrups or elixirs. To formulate as tablets and capsules, the pharmaceutical compositions of the present invention can contain binders such as lactose, saccharose, sorbitol, mannitol, starch, amylopectin, cellulose or gelatin, excipients such as dicalcium phosphate, disintegrants such as corn starch or sweet potato starch, and lubricants such as magnesium stearate, calcium stearate, sodium stearyl fumarate or polyethylene glycol wax. In the case of capsule formulation, in addition to the above-mentioned substances, a liquid carrier such as fatty oil is contained.

The pharmaceutical composition of the present invention can be administered orally or parenterally. For parenteral administration, subcutaneous injection, intravenous injection, intramuscular injection, or intra-thoracic injection can be selected. To prepare the pharmaceutical composition as a formulation for parenteral administration, the composition of the present invention is mixed with a stabilizer or a buffering agent to produce a solution or suspension, which is then formulated as ampoules or vials. The pharmaceutical composition of the present invention can be administered alone or treated together with surgical operation, radio-therapy, hormone therapy, chemo-therapy and biological regulators.

The effective dosage of the active ingredient of the present invention can be determined according to absorptiveness of the active ingredient, inactivation rate, excretion rate, age, gender, health condition and severity of a disease by those in the art.

The present invention also provides a method for secreting an inhibitory material for EMT (epithelial-mesenchymal transition) of cancer cells from macrophages which comprises a step of co-culturing microphages and apoptotic cells.

The present invention also provides a preparation method of a composition for inhibiting cancer migration and invasion comprising a step of co-culturing microphages and apoptotic cells.

The present invention also provides a method for inhibiting EMT (epithelial-mesenchymal transition) of cancer cells by treating conditioned medium (CM) from macrophages exposed to apoptotic cancer cells to cancer cells.

The apoptotic cell above is preferably prepared by irradiating epithelial cancer cells with UV at the wavelength of 100 ∼ 400 nm for 5 ∼ 30 minutes, more preferably at the wavelength of 150 ∼ 350 nm for 5 ∼ 20 minutes and most preferably at the wavelength of 200 ∼ 300 nm for 10 ∼ 15 minutes.

After irradiating the epithelial cancer cells above, the cells can be cultured in RPMI 1604 medium supplemented with 10% FBS in 1 ∼ 10% CO₂ at 30 ∼ 40 °C for 1 ∼ 5 hours.

The culture herein is preferably performed in X-VIVO or serum free DMEM for 20 ∼ 30 hours before the macrophages are stimulated by apoptotic cells. In a preferred embodiment of the present invention, it is more preferred that the cells are cultured in X-VIVO medium for 24 hours to make them serum-deficient. To stimulate the apoptotic cells, the culture medium can be replaced with X-VIVO or serum-free DMEM containing apoptotic cells. The culture preferably continued for 10 ∼ 30 more hours after the medium replacement, and more preferably continued for 15 ∼ 25 hours and most preferably for 18 ∼ 24 hours.

The present invention also provides a health functional food comprising conditioned medium (CM) from macrophages exposed to apoptotic cancer cells as an active ingredient for inhibiting EMT (epithelial-mesenchymal transition), migration and invasion of cancer cells.

The apoptotic cell above is preferably prepared by irradiating epithelial cancer cells with UV at the wavelength of 100 ∼ 400 nm for 5 ∼ 30 minutes, more preferably at the wavelength of 150 ∼ 350 nm for 5 ∼ 20 minutes and most preferably at the wavelength of 200 ∼ 300 nm for 10 ∼ 15 minutes.

After irradiating the epithelial cancer cells above, the cells can be cultured in RPMI 1604 medium supplemented with 10% FBS in 1 ∼ 10% CO₂ at 30 ∼ 40 °C for 1 ∼ 5 hours.

The culture herein is preferably performed in X-VIVO or serum free DMEM for 20 ∼ 30 hours before the macrophages are stimulated by apoptotic cells. To stimulate the apoptotic cells, the culture medium can be replaced with X-VIVO or serum-free DMEM containing apoptotic cells. The culture preferably continued for 10 ∼ 30 more hours after the medium replacement, and more preferably continued for 15 ∼ 25 hours and most preferably for 18 ∼ 24 hours.

The health functional food of the present invention can additionally include various flavors or natural carbohydrates. The natural carbohydrates above can be one of monosaccharides such as glucose and fructose, disaccharides such as maltose and sucrose, polysaccharides such as dextrin and cyclodextrin, and glucose alcohols such as xylitol, sorbitol and erythritol. Besides, natural sweetening agents such as thaumatin and stevia extract, and synthetic sweetening agents such as saccharin and aspartame can be included as a sweetening agent. The content of the natural carbohydrate is preferably 0.01 ∼ 0.04 weight part and more preferably 0.02 ∼ 0.03 weight part in 100 weight part of the health functional food of the present invention.

In addition to the ingredients mentioned above, the health functional food of the present invention can include in a variety of nutrients, vitamins, minerals, flavors, coloring agents, pectic acid and its salts, alginic acid and its salts, organic acid, protective colloidal viscosifiers, pH regulators, stabilizers, antiseptics, glycerin, alcohols, carbonators which used to be added to soda, etc. All the mentioned ingredients can be added singly or together. The mixing ratio of those ingredients does not matter in fact, but in general, each can be added by 0.01 ∼ 0.1 weight part per 100 weight part of the health functional food of the present invention.

The present invention also provides an anticancer adjuvant comprising conditioned medium (CM) from macrophages exposed to apoptotic cancer cells as an active ingredient for inhibiting EMT (epithelial-mesenchymal transition), migration and invasion of cancer cells.

The apoptotic cell above is preferably prepared by irradiating epithelial cancer cells with UV at the wavelength of 100 ∼ 400 nm for 5 ∼ 30 minutes, more preferably at the wavelength of 150 ∼ 350 nm for 5 ∼ 20 minutes and most preferably at the wavelength of 200 ∼ 300 nm for 10 ∼ 15 minutes.

After irradiating the epithelial cancer cells above, the cells can be cultured in RPMI 1604 medium supplemented with 10% FBS in 1 ∼ 10% CO₂ at 30 ∼ 40 °C for 1 ∼ 5 hours.

The culture herein is preferably performed in X-VIVO or serum free DMEM for 20 ∼ 30 hours before the macrophages are stimulated by apoptotic cells. To stimulate the apoptotic cells, the culture medium can be replaced with X-VIVO or serum-free DMEM containing apoptotic cells. The culture preferably continued for 10 ∼ 30 more hours after the medium replacement, and more preferably continued for 15 ∼ 25 hours and most preferably for 18 ∼ 24 hours.

The anticancer adjuvant above can additionally include one or more active ingredients which display the same or similar function to the macrophage and apoptotic cell co-cultured fluid above. The anticancer adjuvant of the present invention can be administered orally or parenterally. For parenteral administration, intraperitoneal injection, intrarectal injection, subcutaneous injection, intravenous injection, intramuscular injection, intrauterine injection, intracerebral injection, or intrathoracic injection can be selected. The anticancer adjuvant of the present invention can be used in general forms of pharmaceutical formulation.

The anticancer adjuvant of the present invention can be administered alone or treated together with surgical operation, radio-therapy, hormone therapy, chemo-therapy and biological regulators.

The dosage of the anticancer adjuvant is 0.0001 ∼ 100 mg/kg per day and preferably 0.001 ∼ 10 mg/kg per day, and administration frequency is once a day or preferably a few times a day. The effective dosage of the anticancer adjuvant can be determined according to weight, age, gender, health condition, diet, administration frequency, administration method, excretion and severity of a disease.

The anticancer adjuvant of the present invention can be administered orally or parenterally. The anticancer adjuvant of the present invention can be prepared for oral or parenteral administration by mixing with generally used diluents or excipients such as fillers, extenders, binders, wetting agents, disintegrating agents and surfactant. Formulations for parenteral administration are sterilized aqueous solutions, water-insoluble excipients, suspensions, emulsions, lyophilized preparations, and suppositories. Water insoluble excipients and suspensions can contain, in addition to the active compound or compounds, propylene glycol, polyethylene glycol, vegetable oil like olive oil, injectable ester like ethylolate, etc. Suppositories can contain, in addition to the active compound or compounds, witepsol, macrogol, tween 61, cacao butter, laurin butter, glycerogelatin, etc.

The present invention also provides a method for preventing or treating cancer comprising a step of administering conditioned medium (CM) from macrophages exposed to apoptotic cancer cells, or apoptotic cancer cells to a subject with cancer.

The present invention also provides a method for inhibiting EMT (epithelial-mesenchymal transition), migration or invasion of cancer cells comprising a step of administering conditioned medium (CM) from macrophages exposed to apoptotic cancer cells, or apoptotic cancer cells to a subject with cancer.

The present invention also provides a method for improving cancer treatment effect comprising a step of administering conditioned medium (CM) from macrophages exposed to apoptotic cancer cells, or apoptotic cancer cells to a subject with cancer.

The present invention also provides a use of conditioned medium (CM) from macrophages exposed to apoptotic cancer cells, or apoptotic cancer cells for the prevention or treatment of cancer.

The present invention also provides a use of conditioned medium (CM) from macrophages exposed to apoptotic cancer cells for inhibiting EMT (epithelial-mesenchymal transition), migration or invasion of cancer cells.

The present invention also provides a use of conditioned medium (CM) from macrophages exposed to apoptotic cancer cells for the preparation of a health functional food for inhibiting migration or invasion of cancer cells.

In addition, the present invention provides a use of conditioned medium (CM) from macrophages exposed to apoptotic cancer cells for the preparation of an anticancer adjuvant for inhibiting EMT (epithelial-mesenchymal transition), migration or invasion of cancer cells.

The present invention suggests through the results of *in vivo* experiments that the direct injection of apoptotic cancer cells can be utilized as a new cell therapy method for suppressing cancer metastasis.

The apoptotic cell above is preferably prepared by irradiating epithelial cancer cells with UV at the wavelength of 100 ∼ 400 nm for 5 ∼ 30 minutes, more preferably at the wavelength of 150 ∼ 350 nm for 5 ∼ 20 minutes and most preferably at the wavelength of 200 ∼ 300 nm for 10 ∼ 15 minutes.

After irradiating the epithelial cancer cells above, the cells can be cultured in RPMI 1604 medium supplemented with 10% FBS in 1 ∼ 10% CO₂ at 30 ∼ 40 °C for 1 ∼ 5 hours.

The culture herein is preferably performed in X-VIVO or serum free DMEM for 20 ∼ 30 hours before the macrophages are stimulated by apoptotic cells. To stimulate the apoptotic cells, the culture medium can be replaced with X-VIVO or serum-free DMEM containing apoptotic cells. The culture preferably continued for 10 ∼ 30 more hours after the medium replacement, and more preferably continued for 15 ∼ 25 hours and most preferably for 18 ∼ 24 hours.

Practical and presently preferred embodiments of the present invention are illustrative as shown in the following Examples.

However, it will be appreciated that those skilled in the art, on consideration of this disclosure, may make modifications and improvements within the spirit and scope of the present invention.

### Example 1: Cell culture

The murine lung adenocarcinoma cell line 344SQ and the human cancer cell lines 344SQ (lung cancer), MDA-MB-231 (breast cancer), COLO320HSR (colon cancer) and PC3 (prostate cancer) were cultured in RPMI 1640 (Media Tech Inc.) supplemented with 10% FBS (fetal bovine serum) and 1% penicillin/streptomycin in a 37°C 5% CO₂ incubator. All the cancer cell lines and RAW 264.7 cells were purchased from ATCC (American Type Culture Collection, Manassas, VA, USA). Mouse RAW 264.7 macrophages (American Type Culture Collection, Manassas, VA, USA) were cultured in DMEM supplemented with 10% FBS and 1% penicillin/streptomycin. To obtain BMDM (mouse bone marrow-derived macrophages) or TAM (M2-like tumor-associated macrophage) cells, bone marrow was extracted from C57BL mouse and then cultured in the medium for BMDM supplemented with 10% FBS and 20% L292 additive for 7 days. To induce each differentiation, the cells were further cultured for 3 days in the medium for BMDM containing 50 ng/ml of IL.

### Example 2: Preparation of conditioned medium (CM)

Mouse macrophages (RAW 264.7) were transferred in a plate at the density of 5 X 10⁵ cells/mℓ, followed by culture in DMEM (Media Tech Inc., Washington, DC, USA) in a 37°C, 5% CO₂ incubator. After overnight culture, serum-free condition was induced in X-VIVO 10 medium for 24 hours before the cells were stimulated. To stimulate the cells, the medium was replaced with another X-VIVO 10 medium containing apoptotic or necrotic cells (1.5 X 10⁶ cells/mℓ). 24 hours later, the supernatant was collected by using a centrifuge, which was used as the conditioned medium for stimulating the target epithelial cells.

To prepare apoptotic cells, epithelial cancer cells were irradiated with UV at the wavelength of 254 nm for 10 minutes, followed by culture in RPMI-1640 supplemented with 10% FBS in a 37°C, 5% CO₂ incubator for 2 hours. In the evaluation of the morphology of cell nucleus using Wright-Giemsa and microscope, it was confirmed that about 70 to 80% of apoptosis was observed in the cells irradiated with UV. The lysed necrotic epithelial cancer cells were obtained by repeated freezing-thawing cycle. Cell apoptosis and necrosis were confirmed by flow cytometric analysis using FACSCalibur system (BD Biosciences) after Annexin V-FITC/propidium iodide (BD Biosciences, San Jose, CA).

### Example 3: Blood sample collection from patients

Lung cancer patients and healthy controls were participated in this experiment after the agreement according to the approval and procedures of Institutional Review Board of Ewha Womans University School of Medicine. Particularly, three healthy controls (1 male, 2 females) and non-small cell lung cancer patients (1 male, 2 females) not treated with anti-cancer agents were participated in the experiment. Peripheral blood mononuclear cells (PBMC) were separated from 20 mℓ of blood sample obtained from each participant by density gradient centrifugation. The purified PBMCs were cultured in RPMI supplemented with 10% human AB serum for 8 days. The differentiation of mononuclear cells into macrophages was confirmed by using anti-F4/80 and confocal microscopy.

### Example 4: Mouse experiment

Animal Care Committee of Ewha Medical Research Institute approved the procedure of this experiment. The management and use of laboratory animals followed NIH (National Institute of Health) guidelines (Care and Use of Laboratory Animals). In this experiment, at least 8 week old mice were used and each group was composed of 15 mice.

### Experimental Example 1: Inhibition of EMT and migration of cancer cells by the conditioned medium

The inhibition of EMT and migration of cancer cells by the conditioned medium obtained from RAW 264.7 cells stimulated by apoptotic 344SQ epithelial cancer cells was investigated.

Particularly, RAW 264.7 cells were stimulated by apoptotic or necrotic 344SQ epithelial cancer cells for 24 hours according to the same manner as described in Example 2. Then, 344SQ cancer epithelial cells were treated with the conditioned medium treated with 10 ng/mℓ of TGF-β1 to induce epithelial-mesenchymal transition (EMT) or the conditioned medium non-treated with TGF-β1. The metastasis changes of the lung adenocarcinoma cell line 344SQ were confirmed by immune-blotting, qPCR (real-time quantitative PCR) and phase-contrast microscopy.

### <1-1> Immuno-blotting

To confirm the expressions of the epithelial and mesenchymal cell markers in the 344SQ epithelial cancer cells of Experimental Example 1 by immune-blotting, cells were lysed in a lysis buffer containing 0.5% Triton-X 100. 10% SDS-PAGE gel was used for the confirmation, and the bands were transferred onto a nitrocellulose membrane. The membrane was blocked with Tris buffer containing 3% BAS at room temperature and then reacted with the primary antibodies shown in Table 1 at room temperature, followed by reaction with the secondary HRP-conjugated antibody.

**[Table 1]**

| Antibody | Manufacturer |
|---|---|
| anti-E-cadherin | ab133597, Abcam, Cambridge, MA, USA |
| N-cadherin | 061, Cell Signaling Technology, Beverly, MA, USA |
| vimentin | NB100-74564, Novus Biologicals, Littleton, CO, USA |
| tubulin | T5168, Sigma-Aldrich, St. Louis, MO, USA |
| Anti-mouse IgG-HRP | sc-2005, Santa Cruz Biotechnology Inc, Finnell Street Dallas, TX, USA |
| Anti-rabbit IgG-HRP | sc-2004, Santa Cruz Biotechnology Inc, Finnell Street Dallas, TX, USA |

As a result, as shown in Figure 1a, when the cells induced with EMT by TGF-β1 were treated with the apoptotic conditioned medium, TGF-β1-induced E-cadherin down-regulation and N-cadherin and vimentin up-regulations were all inhibited (Figure 1a). However, when the cells were treated with the necrotic conditioned medium, TGF-β1-induced EMT was not suppressed. Besides, when 344SQ epithelial cancer cells were directly treated with apoptotic 344SQ epithelial cancer cells, TGF-β1-induced EMT was not suppressed (Figure 1b).

### <1-2> qPCR

Real-time quantitative PCR (qPCR) was performed using *Zeb1, Snai1,* Cdh2 and Vim as the mesenchymal cell markers and Cdh1, Crb3, Cldn3 and Pard6a as the epithelial cell markers in the 344SQ epithelial cancer cells of Experimental Example 1.

Particularly, in order to perform qPCR (Real-Time quantitative PCR), the cells were cultured in the conditioned medium until the confluency reached 80% of a 6-well plate, which were then washed with cold PBS twice. RNA was extracted by using Isol-RNA Lysis Reagent (5PRIME) according to the manufacturer's protocol. The obtained mRNA was reverse-transcribed using iScript™ cDNA Synthesis Kit (BioRad) . For the quantitative PCR, the cDNA product was diluted at the ratio of 1:5, followed by amplification using SYBR Green PCR Master Mix (Applied Biosystems). The reactant was analyzed by using Real-Time PCR System (Applied Biosystems, Step One Plus). The mRNA level was standardized based on mouse HPRT mRNA and human GAPDH mRNA. The primers used in qPCR are as shown in Table 2 below. The test results are shown in Figures, wherein the control (white rod) is presented as a standard and each bar indicates mean ± SEM (n=3). P value was determined by the results of two repeated tests

**[Table 2]**

| murine gene | *forward (5*' → *3*'*)* | *reverse (5*' *→ 3*'*)* |
|---|---|---|
| Zeb1 | *ATTCAGCTACTGTGAGCCCTGC (SEQ. ID. NO: 1)* | *CATTCTGGTCCTCCACAGTGGA (SEQ. ID. NO: 2)* |
| Cdh2 | *CCTCCAGAGTTTACTGCCATGAC (SEQ. ID. NO: 3)* | *CCACCACTGATTCTGTATGCCG (SEQ. ID. NO: 4)* |
| Vim | *GCGTGCGGCTGCTTCAAGAC (SEQ. ID. NO: 5)* | *ATGGCGTCGGCCAGCGAGAA (SEQ. ID. NO: 6)* |
| Cdh1 | *TACGGCGGTGGTGAGGACGA (SEQ. ID. NO: 7)* | *GCCACACGGGGGAGACTTGC (SEQ. ID. NO: 8)* |
| Crb3 | *CGGACCCTTTCACAAATAGCA (SEQ. ID. NO: 9)* | *CGTTGGACTCATCACCTGGG (SEQ. ID. NO: 10)* |
| Cldn3 | *CACCTGACTACCGGGCCTAG (SEQ. ID. NO: 11)* | *GGTTTCTTTGTCCATTCGGCT (SEQ. ID. NO: 12)* |
| Par6b | *TTTCCACCGCCAATCCACTGCT (SEQ. ID. NO: 13)* | *GCTGATGACGATGTGAGGCTTC (SEQ. ID. NO: 14)* |

As a result, as shown in Figure 1c, when the cells induced with EMT by TGF-β1 were treated with the apoptotic conditioned medium, the mRNA expression of the transcription factor known as an EMT signal transduction regulation marker was significantly reduced. However, when the cells were treated with the necrotic conditioned medium, the up-regulation of the transcription factor mRNA mediated by TGF-β was not suppressed (Figure 1).

### <1-3> Cancer migration assay

To investigate the cancer cell migration in the 344SQ epithelial cancer cells of Experimental Example 1, the following experiment was performed using 10 *µ*g/mℓ of fibronectin-coated transwell chambers (Corning Inc) according to the manufacturer's protocol. Briefly, the cells cultured in the conditioned medium prepared in Example 1 at the density of 5 × 10⁴ cells/well for the analysis of cell migration were transferred respectively to the upper chamber wells containing serum-free RPMI medium and to the bottom wells containing RPMI 1640 medium supplemented with 10% FBS, followed by culture at 37°C for 16 hours. After fixing the cells with 4% paraformaldehyde, the non-migrated cells remaining on the upper surface were scratched out. The cells on the bottom surface were stained with 0.05% crystal violet, followed by washing with distilled water. Three microscope fields (10X) were randomly selected and photographed, and the number of cells in each chamber was counted. The cells on the bottom wells were presented by mean ± SEM and the cell migration pattern was visualized by using a phase contrast microscope.

As a result, as shown in Figures 1d and 1e, the retention of the phenotype of cancer cells having mobility and the inhibition of migration of cancer cells were confirmed in the conditioned medium of apoptotic 344SQ epithelial cancer cells (Figure 1).

### Experimental Example 2: Inhibitory effect of the conditioned medium on cancer invasion

The inhibitory effect of the conditioned medium obtained from RAW 264.7 cells stimulated by apoptotic 344SQ epithelial cancer cells on cancer invasion was investigated.

Particularly, the cancer invasion capacity was investigated by qPCR using the cancer stem cell markers. The cancer invasion capacity was also confirmed by phase contrast microscopy and 3D matrigel culture. First, qPCR using the cancer stem cell markers was performed by the same manner as described in Experimental Example <1-2> and the primers used herein are as shown in Table 3 below.

**[Table 3]**

| Marker | *forward (5' → 3')* | *reverse (5' → 3')* |
|---|---|---|
| CD90 | *GTCAGGCTGGTCACCTTCTG (SEQ. ID. NO: 15)* | *AACTCTTGGCACCATGAACC (SEQ. ID. NO: 16)* |
| Oct4 | *TCTTCTGCTTCAGCAGCTTG (SEQ. ID. NO: 17)* | *GTTGGAGAAGGTGGAACCAA (SEQ. ID. NO: 18)* |
| CD44 | *AGAAATGGGTTCAGTGTGGC (SEQ. ID. NO: 19)* | *TTGCTACCACATCAAGCTGC (SEQ. ID. NO: 20)* |
| CD133 | *TAGAGGGAAGTCATTCGGCT (SEQ. ID. NO: 21)* | *CCCAAGATACCTTCAATGCTG (SEQ. ID. NO: 22)* |

The cancer invasion analysis using phase contrast microscopy was performed by the same manner as described in Experimental Example <1-3>.

The cancer cell invasion in 344SQ epithelial cancer cells was analyzed using 200 *µ*g/mℓ of matrigel-coated transwell chambers (Corning Inc) and the cells for invasion analysis which were cultured at the density of 2 × 10⁵ cells/well according to the manufacturer's protocol. Particularly, the basic 3D matrigel culture was performed with 344SQ epithelial cancer cells (5000 cells/well) layered on the solid Growth Factor Reduced Matrigel (Corning Inc) as in RPMI1640 supplemented with 10% FBS and 2% matrigel. The culture medium was replaced every 2 ∼ 3 days for a week. One week later, 10 ng/mℓ of TGF-β1, 2% matrigel and the apoptotic conditioned medium were treated to cancer cells, followed by culture for 3 days. The phase contrast images were analyzed by using Eclipse TE-300 microscope (Nikon).

As a result, as shown in Figure 2, when the apoptotic conditioned medium was treated to the cells induced with EMT by TGF-β1, the mRNA expressions of the cancer stem cell markers CD90, Oct4, CD44 and CD133 were significantly reduced (Figure 2a). From the observation with a phase contrast microscope, it was confirmed that the number of cells invaded was significantly reduced compared with the control (Figure 2b). When 344SQ epithelial cancer cells were cultured in matrigel, the cell polarity was strengthened (Figure 2c).

### Experimental Example 3: Inhibitory effect of the apoptotic conditioned medium on EMT-induced cancer metastasis and invasion in human non-small cell lung carcinoma (NSCLS) cell line (A549)

The inhibitory effect of the apoptotic conditioned medium on EMT (epithelial-mesenchymal transition), migration and invasion of cancer cells in the human A549 cell line was investigated.

Particularly, immune-blotting, qPCR (Real-Time quantitative PCR) and phase-contrast microscopy were performed by the same manner as described in Experimental Example 1, in order to investigate EMT (epithelial-mesenchymal transition), migration and invasion of cancer cells. The primers used for qPCR are as shown in Table 4 below.

**[Table 4]**

| Human gene | Forward (5' → 3') | Reverse (5' → 3') |
|---|---|---|
| Snai1 | *TGCCCTCAAGATGCACATCCGA (SEQ. ID. NO: 23)* | *GGGACAGGAGAAGGGCTTCTC (SEQ. ID. NO: 24)* |
| Cdh2 | *CCTCCAGAGTTTACTGCCATGAC (SEQ. ID. NO: 25)* | *CCTCCAGAGTTTACTGCCATGAC (SEQ. ID. NO: 26)* |
| Vim | *AGGCAAAGCAGGAGTCCACTGA (SEQ. ID. NO: 27)* | *ATCTGGCGTTCCAGGGACTCAT (SEQ. ID. NO: 28)* |
| Cdh1 | *GCCTCCTGAAAAGAGAGTGGAAG (SEQ. ID. NO: 29)* | *TGGCAGTGTCTCTCCAAATCCG (SEQ. ID. NO: 30)* |
| Par6b | *GTTTCAACGGCCAATCCACTGC (SEQ. ID. NO: 31)* | *CTATGGTTGTCAGGACGCAATAC (SEQ. ID. NO: 32)* |
| OCLN | *ATGGCAAAGTGAATGACAAGCGG (SEQ. ID. NO: 33)* | *CTGTAACGAGGCTGCCTGAAGT (SEQ. ID. NO: 34)* |
| ALDH1A | *CCACTCACTGAATCATGCCA (SEQ. ID. NO: 35)* | *GCACGCCAGACTTACCTGTC (SEQ. ID. NO: 36)* |
| CD44 | *CACGTGGAATACACCTGCAA (SEQ. ID. NO: 37)* | *GACAAGTTTTGGTGGCACG (SEQ. ID. NO: 38)* |
| CD90 | *GGGAGACCTGCAAGACTGTT (SEQ. ID. NO: 39)* | *CGGAAGACCCCAGTCCA (SEQ. ID. NO: 40)* |

As a result, as shown in Figure 3 and Figure 4, when the apoptotic conditioned medium was treated to the cells induced with EMT by TGF-β1, the expression of E-cadherin was decreased and the up-regulations of N-cadherin and vimentin were reversed (Figure 3a). It was also confirmed that the enhanced expression of the mesenchymal marker mRNA and the decreased expression of the epithelial marker mRNA were reversed by treatment with the apoptotic conditioned medium (Figure 3c). As shown in Figure 3c, when the apoptotic conditioned medium was treated to the cells induced with EMT by TGF-β1, the expression of mRNA of the transcription factor known as the EMT signal transduction regulation marker was significantly reduced (Figure 3). As shown in Figures 3d and 3e, the retention of the phenotype of cancer cells having mobility and the inhibition of migration of cancer cells were confirmed in the conditioned medium of apoptotic A549 epithelial cancer cells (Figure 3). As shown in Figure 4, when the apoptotic conditioned medium was treated to the cells induced with EMT by TGF-β1, the expressions of the cancer stem cell markers ALDH1A, CD44 and CD90 mRNAs were significantly reduced (Figure 4a). It was also confirmed through phase contrast microscopy that the number of infiltrated cells was significantly reduced compared to the control group (Figure 4b).

### Experimental Example 4: Effect of the apoptotic conditioned medium on the cellular proliferative activity in lung cancer cells

The inhibitory effect of the apoptotic conditioned medium on EMT, migration and invasion of cancer cells was confirmed in Experimental Example 1 ∼ Experimental Example 3 above. So, the present inventors further investigated whether or not the above effect could also affect the cellular proliferative activity.

Particularly, lung cancer cells (344Q and A549, 1 × 10⁴ cells/well) were distributed in a 96-well culture plate, followed by culture in RPMI1640 medium supplemented with 10% FBS for 1 day. The culture medium was replaced with the conditioned medium supplemented or not supplemented with TGF-β1, followed by further culture for 48 hours. Then, 3-(4,5-dimethylthiazol-2-thiazolyl)-2,5 diphenyl tetrazolium bromide (MTT) assay (Sigma-Aldrich, M5655) was performed at 37°C for 4 hours. The cells were lysed in dimethyl sulfoxide (100 *µ*ℓ/well), followed by culture in a shaker at room temperature for 30 minutes in the dark. OD₅₇₀ was measured using a microplate reader (Molecular devices, Versa max) to investigate cell proliferation.

As a result, as shown in Figure 5, the apoptotic conditioned medium did not affect the proliferation of lung cancer cells at all (Figure 5).

### Experimental Example 5: Anti-EMT effect of the apoptotic conditioned medium in various human cancer cell lines

To investigate the effect of the conditioned medium in various cancer cell lines, the following experiment was performed.

Particularly, MDA-MB-231 (breast cancer), COLO320HSR (colon cancer) and PC3 (prostate cancer) cells were treated with the apoptotic conditioned medium (Apo CM) or the necrotic conditioned medium (Nec CM) obtained from RAW264.7 cells treated or non-treated with TGF-β1. Then, immune-blotting was performed by the same manner as described in Example <1-1>.

As a result, as shown in Figure 6, when the apoptotic conditioned medium was treated to the cells induced with EMT by TGF-β1, the down-regulation of E-cadherin and the up-regulations of N-cadherin and vimentin caused by TGF-β1 were inhibited in all of the breast cancer cells, colon cancer cells and prostate cancer cells (Figure 6). However, the treatment of the necrotic conditioned medium did not suppress EMT induced by TGF-β1 (Figure 6).

### Experimental Example 6: Anti-EMT effect of the conditioned medium conditioned with various macrophages

The EMT inhibitory effect of the conditioned media with various macrophages having different origins, in addition to RAW 264.7 macrophages was investigated.

Particularly, the conditioned medium obtained from mouse bone marrow-derived macrophages (BMDM) or M2-like tumor-associated macrophage (TAM) stimulated by apoptotic 344SQ epithelial cells was treated to 344SQ epithelial cells for 48 hours. Then, immune-blotting was performed to investigate the changes of cadherin and vimentin according to the presence or absence of TGF-β1.

As a result, as shown in Figure 7, when the apoptotic conditioned medium obtained from BMDM or TAM was treated to the cells induced with EMT by TGF-β1, the TGF-β1-induced down-regulation of E-cadherin and the TGF-β1-induced up-regulations of N-cadherin and vimentin were all inhibited (Figures 7a and 7b).

The conditioned medium obtained from human blood monocyte-derived macrophages of healthy donors or human blood monocyte-derived macrophages of lung cancer patients stimulated by apoptotic or necrotic A549 epithelial cancer cells (see Experimental Example 3) was treated to A549 epithelial cancer cells in the presence or absence of 10 ng/mℓ of TGF-β1, followed by the investigation by the same manner as described above.

As a result, as shown in Figure 8, when the conditioned medium obtained from human blood monocyte-derived macrophages of healthy donors or human blood monocyte-derived macrophages of lung cancer patients stimulated by apoptotic or necrotic A549 epithelial cancer cells was treated to the cells induced with EMT by TGF-β1, the TGF-β1-induced down-regulation of E-cadherin and the TGF-β1-induced up-regulations of N-cadherin and vimentin were all inhibited in the apoptotic conditioned medium. However, the TGF-β1-induced EMT was not suppressed in the necrotic conditioned medium (Figures 8a and 8b).

### Experimental Example 7: Confirmation of anti-EMT mechanism of the apoptotic conditioned medium

To investigate anti-EMT mechanism of the apoptotic conditioned medium, the following experiment was performed.

First, 344SQ epithelial cancer cells were treated with 10 ng/mℓ of TGF-β1 over the various treatment time. Then, immune-blotting was performed to analyze Smad2, Samd3, ERK1/2, p38 and AKT, the signaling proteins activated by TGF-β1.

As a result, as shown in Figure 9, it was confirmed that the phosphorylation of Smad2, smad3, ERK1/2, p38 and AKT was induced by TGF-β1 (Figure 9a).

Next, 344SQ epithelial cells were treated with the conditioned medium obtained from RAW 264.7 cells stimulated by apoptotic 344SQ epithelial cancer cells and 10 ng/mℓ of TGF-β1. Then, immune-blotting was performed with the cell lysis obtained over the time which displayed a significant effect in order to investigate the signaling proteins.

As a result, as shown in Figure 9, when the apoptotic conditioned medium was treated thereto, the TGF-β1-induced phosphorylation of Smad2, Smad3 and ERK1/2 was not changed. In the meantime, the phosphorylation of p38 and AKT was efficiently inhibited by the long time treatment (Figures 9b, 9c, 9d, 9e and 9f). The results above indicate that the apoptotic conditioned medium of the present invention can inhibit EMT independently from TGF-β1-induced Smad signaling.

### Experimental Example 8: PPARγ-dependent up-regulation of PTEN in macrophages stimulated by apoptotic cancer cells

To investigate whether or not the expression of PPARγ or PTEN mRNA was increased in RAW 264.7 cells stimulated by apoptotic or necrotic 344SQ epithelial cancer cells, qPCR and immune-blotting were performed by the same manner as described in Experimental Examples <1-1> and <1-2>.

As a result, as shown in Figure 10, the expressions of PPARγ and PTEN mRNAs and proteins were increased in RAW 264.7 cells stimulated by apoptotic 344SQ epithelial cancer cells (Figures 10a ∼ 10e). When the monocyte-derived macrophages originated from healthy donors or lung cancer patients were stimulated by apoptotic A549 epithelial cancer cells, the up-regulation of PTEN mRNA was also observed (Figure 10f).

As shown in Figure 11, when the PPARγ inhibitor GW9662 was added to the cells one hour before the stimulation with apoptotic 344SQ epithelial cancer cells, the up-regulations of PTEN mRNA and protein induced by apoptotic 344SQ epithelial cancer cells were suppressed (Figures 11a ∼ 11c). It was also confirmed that the PPARγ activity increased by the stimulation with apoptotic 344SQ epithelial cancer cells was suppressed by GW9662 (Figure 11c). When PPARγ siRNA was introduced, the up-regulations of PPARγ and PTEN proteins by the stimulation of apoptotic 344SQ epithelial cancer cells were suppressed in RAW 264.7 cells (Figure 11d). The up-regulation of PTEN protein induced by the stimulation of apoptotic 344SQ epithelial cancer cells in BMDM (mouse bone marrow-derived macrophages) and M2 type BMDM was suppressed by the addition of GW9662 (Figures 11e and 11f).

**[Table 5]**

| siRNA | sense(5' → 3') | antisense (5' → 3') |
|---|---|---|
| PPARγ | *AGUAUGGUGUCCAUGAGAU (SEQ. ID. NO: 41)* | *AUCUCAUGGACACCAUACU (SEQ. ID. NO: 42)* |
| PTEN | *CAGGAAUGAACCAUCUACA (SEQ. ID. NO: 43)* | *UGUAGAUGGUUCAUUCCUG (SEQ. ID. NO: 44)* |
| L-PGDS | *CAACUAUGACGAGUACGCUCU GCUA (SEQ. ID. NO: 45)* | *GACUUCCGCAUGGCCACCCUCU ACA (SEQ. ID. NO: 46)* |

### Experimental Example 9: Involvement of PPARγ in anti-EMT mechanism

To investigate whether or not the increased PPARγ signal caused by the stimulation of apoptotic epithelial cancer cells in macrophages was involved in anti-EMT mechanism in epithelial cancer cells, the following experiment was performed.

RAW 264.7 cells were pretreated with the PPARγ inhibitor GW9662 and stimulated by apoptotic 344SQ cells, from which the conditioned medium was obtained. 344SQ epithelial cancer cells were treated with the conditioned medium along with 10 ng/mℓ of TGF-β1, followed by culture. After 48 hours of culture, the changes of cell morphology were confirmed by phase contrast microscopy and immune-blotting was performed.

As a result, as shown in Figure 12, spindle cells were decreased and the anti-EMT effect (Figure 12a) and the inhibition of the down-regulation of E-cadherin and the up-regulations of N-cadherin and vimentin were reversed (Figures 12a and 12b). Once the related transcription factors *Snai1* and *Zeb1* mRNAs were down-regulated but later on they were up-regulated when RAW 264.7 cells were pre-treated with GW9662 (Figure 12c). In the meantime, the decreased invading cells were increased when RAW 264.7 cells were pre-treated with GW9662 (Figure 12d).

### Experimental Example 10: Confirmation of PPARγ-dependent PTEN secretion in macrophages stimulated by apoptotic cancer cells

To investigate whether or not the PPARγ-dependent PTEN secretion in macrophages stimulated by apoptotic cancer cells was induced in the form of exosomes, the following experiment was performed.

For transmission electron microscopy (TEM), RAW 264.7 cells stimulated by apoptotic 344SQ epithelial cancer cells were fixed with 2.5% glutaraldehyde (in 0.1 M phosphate) for 2 hours, which were then washed with PBS three times for 10 minutes for each. The prepared block was additionally fixed with 1% osmium tetroxide. Every time the cells were washed for 10 minutes twice, ethanol was used with changing their concentrations (60, 70, 80, 90, 95 and 100) for continuously drying. The samples were placed in propylene oxide for 2 changes every 15 min. At this time, the ratio of propylene oxide and epoxy resin (Polysciences Inc) was 2:1, 1:1 and 1:2 and the samples were places in each mixture for one hour. One hour later, the mixture having the ratio of 1:2 was replaced with 100% Epon medium for 30 minutes. The region for observation was selected from the area stained with toluidine blue and of thickness of 1 µm. The sector of about 60 to 70 nm was generated using a diamond knife of ultramicrotome (Reichert-Jung USA). The samples were dried for about 20 minutes, followed by staining with uranyl acetate and lead citrate. The samples were observed under transmission electron microscope (TEM, H-7650; Hitachi). At this time, the accelerating current was 80 kV, and the results were analyzed using iTEM software (Olympus).

For the immunoprecipitation for detection of PTEN, the medium was diluted in 2X exosome lysis buffer (4% SDS, 2% Triton X100, 0.1 M Tris pH 7.4 and 2X protease inhibitor) at the volume ratio of 1:1, which was dissolved at 4 °C for one hour. The medium-lysis buffer mixture was filtered with 0.22 micron filter and the filtrate was distributed in a 15 ml tube, to which 25 µl of anti-PTEN (138G6, Cell Signaling) was added, followed by stirring at 4 °C for 4 hours. The reactant was precipitated by using 100 µl (50% slurry) of Protein A/G Sepharose (Bio Vision Inc). The mixture attached with pull-down bead was transferred into a new tube, followed by washing with IP washing buffer (25 ml HEPES pH 7.4, 1M NaCl, 1 ml EDTA, 0.5% Triton X-100) several times.

In addition, for the separation of exosomes through centrifugation, the conditioned medium was obtained from RAW 264.7 cells stimulated by apoptotic 344SQ epithelial cancer cells and centrifuged serially at 200 g, 20,000 g and 100,000 g by using an ultracentrifuge (Optima L-100K, Beckman Coulter Inc., Brea, CA, USA). The exosome pellet was washed with cold PBS containing a protease inhibitor, followed by centrifugation at 100,000 for 70 minutes. For immune-blotting, the exosome pellet was resuspended in RIPA buffer containing a protease inhibitor.

As a result, as shown in Figure 13, microvesicles were observed in RAW 264.7 cells stimulated by apoptotic 344SQ epithelial cancer cells (Figure 13a).

It was also confirmed by Western blotting that PTEN was clearly observed in BMDM cell lysate wherein PTEN expression was induced by apoptotic 344SQ cells (Figure 13b, left). The PTEN protein expression was confirmed by using SDS-PAGE in the non-reducing condition in the conditioned medium after immunoprecipitation (Figure 13b, right, down arrow). Exosomes were separated from the conditioned medium through step-wise ultracentrifugation. Immunoblotting was performed using PTEN antibody and the exosome marker CD63 antibody. As a result, PTEN protein was increased in the apoptotic conditioned medium without changing CD63. However, the PTEN expression was inhibited by the pre-treatment of GW9662 (Figure 13c). The results above indicate that the PTEN protein increased by PPARγ activated by the stimulation with 344SQ cells in macrophages was inserted in exosomes and secreted.

### Experimental Example 11: Changes of signal, cell polarity and EMT in PTEN-introduced cells

To investigate the changes of cell signal, cell polarity and EMT in 344SQ epithelial cancer cells introduced with PTEN, the conditioned medium and TGF-β1 (10 ng/mℓ) were added to 344SQ epithelial cancer cells, followed by qPCR and immune-blotting.

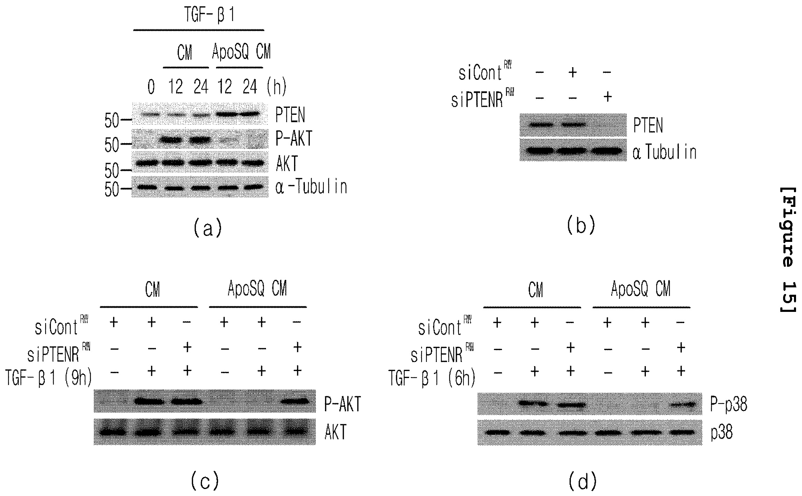

As a result, as shown in Figures 14 and 15, the expression of PTEN was increased rapidly by the conditioned medium and even 24 hours later the expression was still high. However, the basal AKT phosphorylation started decreasing about 6 hours later (Figure 14a). The PTEN mRNA expression was not changed by the addition or absence of TGF-β1. However, as the dilution ratio of the conditioned medium was increased, the expression of PTEN protein was decreased (Figures 14b and 14c). In addition, TGF-β1 did not affect the PTEN protein expression for 24 hours but after the first 24 hours, it reduced the protein expression (Figure 14d). The TGF-β1-induced AKT phosphorylation was suppressed for up to 24 hours when the conditioned medium was treated along with TGF-β1 (Figure 14a). In the cells treated with the conditioned medium obtained from RAW 264.7 cells wherein the PTEN expression was suppressed by treating PTEN siRNA (Figure 15b), the TGF-β1-induced AKT or p38 MAP kinase phosphorylation did not decreased (Figures 15c and 15d). The results above indicate that the PTEN introduced into cells affected the basal AKT phosphorylation and the TGF-β1-induced non-Smad signal.

### Experimental Example 12: Confirmation of cell polarity maintenance effect in PTEN-introduced cells

The following experiment was performed to investigate whether or not PTEN aggregated toward cell membrane in order to maintain cell polarity when the conditioned medium was treated to the cells.

Cell membrane fractionation analysis was performed using Memper™ Eukaryotic Membrane Protein Extraction Reagent Kit according to the manufacturer's protocol.

For immunofluorescence assay, the 344SQ epithelial cells treated above were washed with cold PBS twice, followed by fixing in 4% paraformaldehyde at room temperature for 8 minutes. The cells were washed with PBS three times for 5 minutes per each washing, followed by treating 0.5% Triton X-100 (in PBS) at room temperature for 5 minutes. Then, the blocking solution containing 5% BSA and 5% goat serum was added to the cells, followed by reaction at room temperature. The primary antibody was added thereto at the ratio of 1:500, followed by reaction at 4 °C for overnight. The cells were washed with PBS, to which the secondary antibody was added, followed by reaction at room temperature for 1 hour. Upon completion of the reaction, the cells were washed with PBS three times for 5 minutes per each washing. For staining 344SQ epithelial cancer cells on 3D matrigel, the cells were fixed at room temperature for 30 minutes. The fixed cells were washed with IF-Wash buffer (0.05% NaN3, 0.1% BSA, 0.2% Triton X-100 and 0.05% Tween-20 in PBS), to which the blocking solution containing 5% BSA was added, leading to the pre-treatment at room temperature. The primary antibody diluted in the blocking solution at the ratio of 1:500 was added thereto, followed by reaction at 4°C for overnight. The cells were washed and then added with the secondary antibody diluted in the blocking solution at the ratio of 1:500. The cells were reacted at room temperature for 1 hour, and washed with PBS three times for 5 minutes per each washing. The cells were then treated with VECTASHIELD mounting medium, and observed under a confocal microscope (Carl Zeiss LSM 800) .

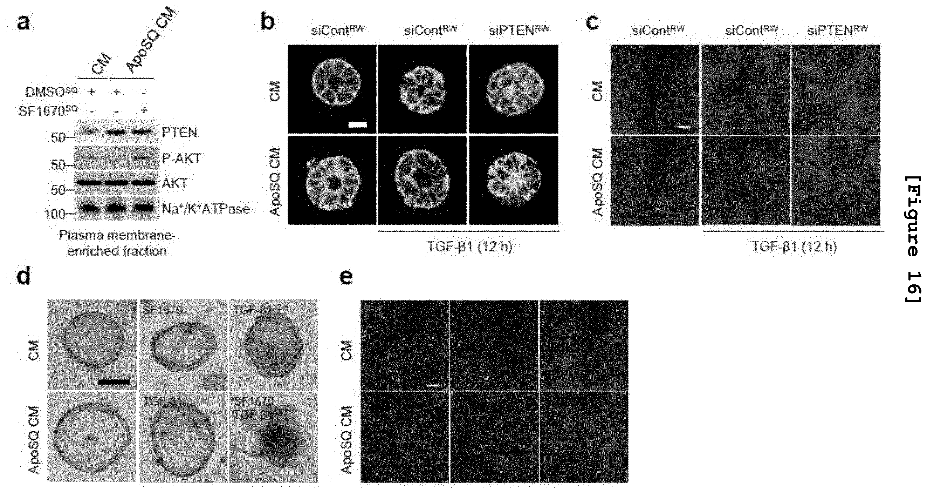

As a result, as shown in Figure 16, when the conditioned medium was treated to 344SQ epithelial cancer cells for 12 hours, the PTEN protein expression was increased on the cell membrane region containing Na⁺/K⁺ ATPase richly, suggesting that PTEN was migrating toward the cell membrane. When the PTEN selective inhibitor SF1670 was treated, the reduced AKT phosphorylation was increased again (Figure 16a). The results above indicate that the gathered PTEN was functioning as an AKT activation inhibitor.

Cell polarity was observed using a confocal microscope. In the 344SQ epithelial cancer cells treated with the conditioned medium obtained from RAW 264.7 cells introduced with control siRNA, the cell polarity disruption caused by TGF-β1 was inhibited. However, in the cells treated with the conditioned medium obtained from RAW 254.7 cells introduced with PTEN siRNA, the cell polarity disruption was observed (Figure 16b). In addition, in the cells treated with the conditioned medium obtained from RAW 264.7 cells introduced with control siRNA, the intercellular contact disruption induced by TGF-β1 was not observed. However, in the cells treated with the conditioned medium obtained from RAW 264.7 cells introduced with PTEN siRNA, the intercellular contact disruption was observed (Figure 16c). Similarly, when SF1670 was treated to 344SQ cells, the cell polarity maintenance and the intercellular contact disruption inhibition effect were reversed by the treatment of the conditioned medium, confirmed by phase contrast images of cells cultured in 3D matrigel and staining photographs of cell clusters (Figures 16d and 16e). The results above indicate that the PTEN introduced in cells inhibited the intercellular contact disruption so that it was involved in the maintenance of cell polarity and cell integrity of epithelial cancer cells.

### Experimental Example 13: Confirmation of anti-EMT inhibition in PTEN-introduced cells

To investigate whether or not the PTEN introduced into cells was involved in the anti-EMT and anti-metastasis activity when the conditioned medium was treated to the cells, 344SQ epithelial cancer cells were treated with the conditioned medium obtained from RAW 264.7 cells introduced with PTEN siRNA along with 10 ng/mℓ of TGF-β1, followed by qPCR and immune-blotting.

As a result, as shown in Figures 17 and 18, the typical anti-EMT effect such as the inhibition of the down-regulation of E-cadherin and the inhibition of the up-regulations of N-cadherin and vimentin was reversed and therefore EMT was induced again (Figure 17a). In addition, the related transcription factors *Snai1* and *Zeb1* mRNAs were up-regulated and accordingly the number of invading cells was increased (Figures 17b and 17c). When the PTEN selective inhibitor SF1670 was treated to 344SQ epithelial cells, the same results were observed as the above (Figures 18a ∼ 18d). The results above indicate that the introduced PTEN or PTEN signal was involved in the anti-EMT mechanism.

### Experimental Example 14: Involvement of PPARγ ligand secreted in macrophages stimulated by apoptotic cancer cells in anti-EMT mechanism

It is known that apoptotic cells stimulate the production of 15-lipoxygenase-dependent 15-hydroxyeicosatetraenoic acid (HETE), lipoxin A4 and PGD₂ synthase-dependent 15-deoxy-Δ12,14-prostaglandin J₂ (15d-PGJ₂) known as PPARγ ligands. Therefore, the present inventors performed the following experiment to investigate the existence of PPARγ ligand in the conditioned medium.

As a result, as shown in Figure 19, it was confirmed that the contents of 15-HETE, lipoxin A4 and 15d-PGJ₂ were increased in the apoptotic conditioned medium, and that the ligands were not increased in the necrotic conditioned medium (Figures 19a ∼ 19c). The results above indicate that the ligand introduced into cells via paracrine manner accelerated the transcription activity of PPARγ on the PTEN promoter.

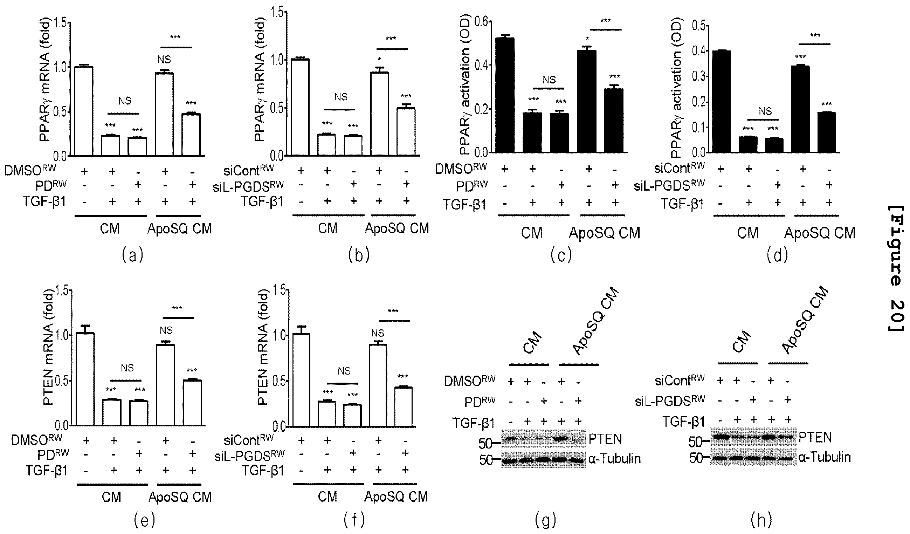

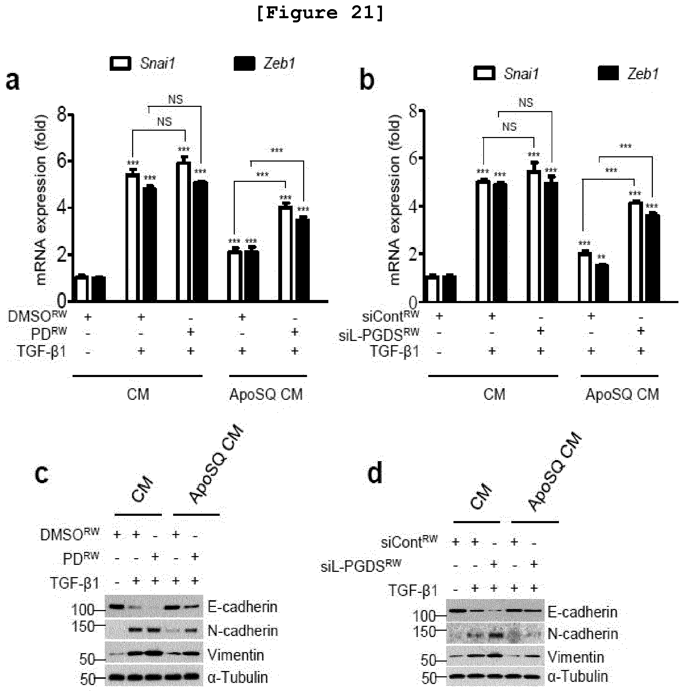

To investigate whether or not the secreted PPARγ ligand was involved in the increase of PPARγ activity in 344SQ cancer cells, the 15-lipoxygenase selective inhibitor PD146176 and the lipocalin-type PGDS specific siRNA were treated to RAW 264.7 cells. As a result, when apoptotic 344SQ cells were stimulated by the conditions above, the contents of 15-HETE, lipoxin A4, PGD₂ and 15d-PGJ₂ were significantly reduced (Figures 19d ∼ 19g). In particular, when 344SQ cancer cells were treated with the conditioned medium wherein PPARγ ligand was reduced, the reversing effect of TGF-β1-induced inhibition of PPARγ mRNA and its activity as well as PTEN mRNA and protein was reduced (Figures 20a ∼ 20h). Consistently, the anti-EMT mechanism was not efficiently working, either (Figures 21a ∼ 21d). The results above indicate that the ligands secreted in macrophages were introduced in 344SQ cancer cells in the condition that they could not be able to affect those macrophages and then involved in the anti-EMT mechanism through the PPARγ and PTEN signal activation.

### Experimental Example 15: Confirmation of anti-EMT effect in cancer cells treated with PPARγ ligand

To investigate whether or not the PPARγ ligands such as 15-HETE, lipoxin A4 and 15d-PGJ₂ of Experimental Example 14 were involved in the anti-EMT mechanism through the activation of PPARγ and PTEN mechanism, the basal concentrations (80, 73 and 73 pg/ml) and the stimulation concentrations (258, 442 and 226 pg/ml) of those ligands were directly added to 344SQ epithelial cancer cells along with TGF-β1, followed by qPCR and immune-blotting.

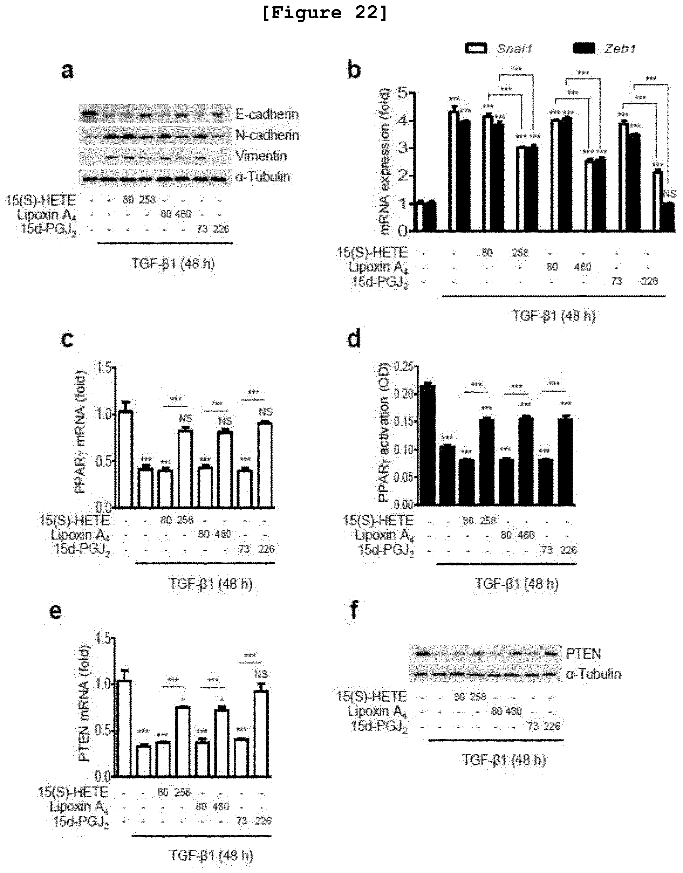

As a result, as shown in Figure 22, no significant effect was observed at the basal concentrations of PPARγ ligands. However, the TGF-β1-induced EMT was inhibited by the treatment of PPARγ ligand at the stimulation concentrations (Figures 22a and 22b). The TGF-β1-induced inhibition of PPARγ mRNA and activity and PTEN mRNA and protein were reversed by the treatment of PPARγ ligand at the basal concentrations (Figures 22c ∼ 22f).

### Experimental Example 16: In vivo investigation of anti-metastasis effect of apoptotic cancer cells in mice

To investigate the effect of apoptotic 344SQ epithelial cancer cells on cancer metastasis in real animals (*in vivo*), the following experiment was performed.

First, 344SQ epithelial cancer cells (approximately 1 x 10⁶ cells) were subcutaneously injected in the right side of the abdomen of the mouse (syngeneic (129/Sn), immunocompetent mouse). 2 days later, the apoptotic 344SQ cells (approximately 1 x 10⁷ cells) prepared in Example 2 and PBS (control, 100 *µ*ℓ) were subcutaneously injected in the skin lesions of the mouse (n= 15). The tumor growth was monitored every day for 6 weeks. Then, each mouse was autopsied to analyze the changes of the primary tumor (Figure 23a) .

As a result, as shown in Figure 23, no significant reductions in tumor weight and number of nodules in the lungs were observed in mice injected with apoptotic epithelial cancer cells (Figures 23b and 23c). However, the ratio of visually identifiable metastasis (Met) was decreased (Figure 23d). In Figure 23e, the tumor nodules observed in the lungs of the control mice were not observed in the mice treated with apoptotic 344SQ cells (Figure 23e).

### Experimental Example 17: Increase of PPARγ and PTEN expressions by apoptotic cancer cells in mice

To investigate the changes of PPARγ and PTEN expression patterns in the primary tumor of the test animal injected with apoptotic 344SQ epithelial cancer cells, the following experiment was performed.

344SQ epithelial cancer cells were subcutaneously injected in the mice by the same manner as described in Experimental Example 16. 2 days later, apoptotic 344SQ epithelial cancer cells or PBS were subcutaneously injected in the mice (n=15). Six weeks later, the mice were autopsied and qPCR, immunoblotting and immunohistochemistry were performed

For immunohistochemistry, the tumor tissues fixed in paraffin or the frozen skin tissues thawed at room temperature for 30 minutes were used. The tissue samples were washed with IF-Wash buffer (0.05% NaN3, 0.1% BSA, 0.2% Triton X-100 and 0.05% Tween-20 in PBS). Then, the samples were blocked with a blocking solution containing 5% BSA and mouse IgG blocking reagent (MKB-2213) at room temperature. The samples were reacted with the primary antibody diluted in the blocking solution at the ratio of 1:100 at 4 °C for overnight. Then, the samples were washed with PBS three times for 5 minutes per each washing. The samples were treated with VECTASHIELD Mounting Medium and the tissues were observed under a confocal microscope (Carl Zeiss LSM 800).

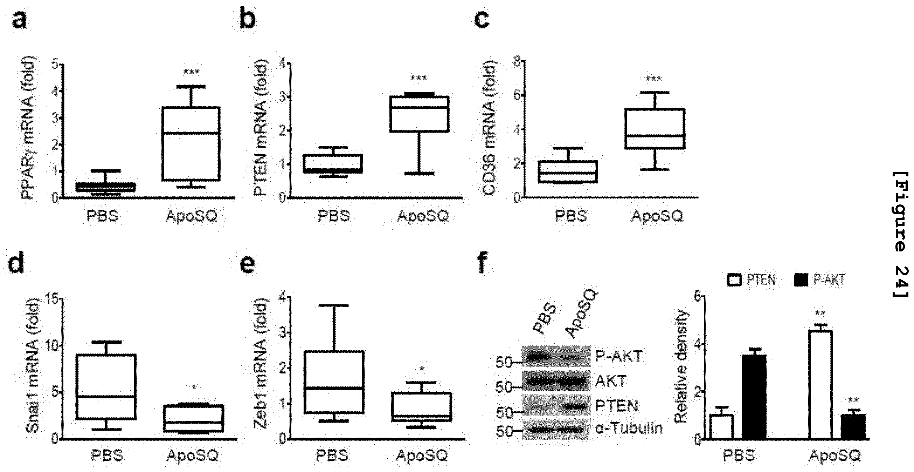

As a result, as shown in Figure 24, in the primary tumor of the mouse injected with apoptotic 344SQ epithelial cancer cells, PPARγ, PTEN and CD36 mRNAs were all increased, but *Snai1* and *Zeb1* mRNAs were reduced (Figure 24a ∼ 24e). The PTEN protein expression was increased but the AKT phosphorylation was reduced (Figure 24f).

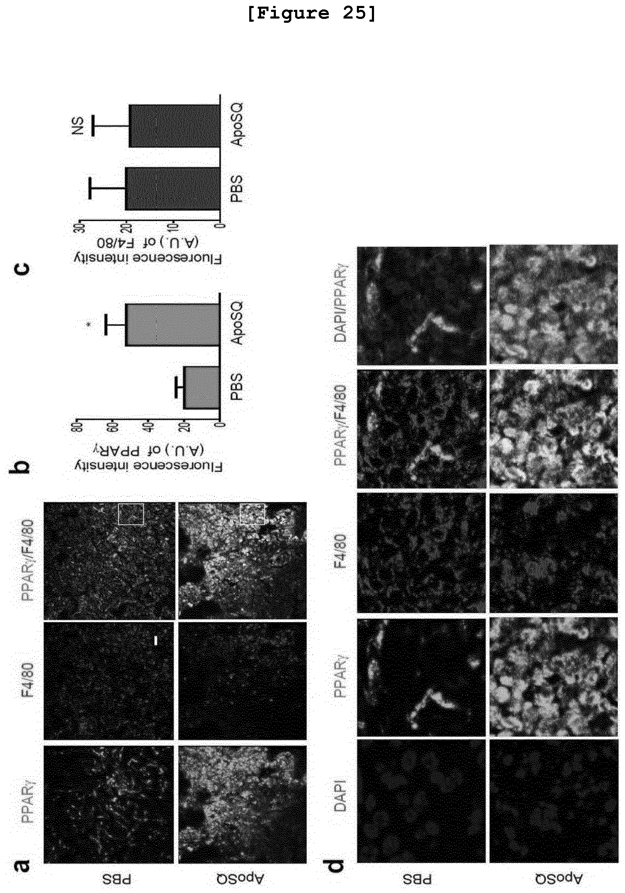

When the tumor was immunohistochemically stained, the up-regulations of PPARγ (Figures 25a and 25b, green), PTEN (Figures 26a and 26b,green) and CD36 (Figures 26d and 26e, red) were observed. In addition, after the injection of apoptotic cancer cells in F4/80 positive cells (red), there was no change in red fluorescence (Figure 25c) and the expressions of PPARγ, PTEN and CD36 were significantly increased (Figures 25d, 26c and 26f). It was also confirmed that PPARγ, PTEN and CD36 were induced in tumor invasive macrophages by apoptotic cells.

Those skilled in the art will appreciate that the conceptions and specific embodiments disclosed in the foregoing description may be readily utilized as a basis for modifying or designing other embodiments for carrying out the same purposes of the present invention. Those skilled in the art will also appreciate that such equivalent embodiments do not depart from the spirit and scope of the invention as set forth in the appended Claims.

## Claims

1. A pharmaceutical composition for the inhibition of EMT (epithelial-mesenchymal transition), migration and invasion of cancer cells comprising conditioned medium (CM) from macrophages exposed to apoptotic cancer cells.

2. The pharmaceutical composition for the inhibition of EMT (epithelial-mesenchymal transition), migration and invasion of cancer cells according to claim 1, wherein the apoptotic cells are prepared by irradiating cancer cells with UV.

3. The pharmaceutical composition for the inhibition of EMT (epithelial-mesenchymal transition), migration and invasion of cancer cells according to claim 2, wherein the apoptotic cells are prepared by irradiating epithelial cancer cells with UV at the wavelength of 100 ∼ 400 nm for 5 ∼ 30 minutes, followed by culture for 1 ∼ 5 hours.

4. The pharmaceutical composition for the inhibition of EMT (epithelial-mesenchymal transition), migration and invasion of cancer cells according to claim 1, wherein the apoptotic cells are 344SQ cells and A549 cells.

5. The pharmaceutical composition for the inhibition of EMT (epithelial-mesenchymal transition), migration and invasion of cancer cells according to claim 1, wherein the culture is performed at 34 ∼ 38 °C for 15 ∼ 25 hours.

6. The pharmaceutical composition for the inhibition of EMT (epithelial-mesenchymal transition), migration and invasion of cancer cells according to claim 1, wherein the culture fluid inhibits EMT (epithelial-mesenchymal transition), the pre-stage of metastasis.

7. The pharmaceutical composition for the inhibition of EMT (epithelial-mesenchymal transition), migration and invasion of cancer cells according to claim 1, wherein the culture fluid inhibits the early stage of cancer metastasis and infiltration.

8. The pharmaceutical composition for the inhibition of EMT (epithelial-mesenchymal transition), migration and invasion of cancer cells according to claim 1, wherein the cancer is selected from the group consisting of lung cancer, breast cancer, colon cancer and prostate cancer.

9. The pharmaceutical composition for the inhibition of EMT (epithelial-mesenchymal transition), migration and invasion of cancer cells according to claim 8, wherein the lung cancer is non-small cell lung cancer.

10. A method for secreting an inhibitory material for EMT (epithelial-mesenchymal transition) of cancer cells from macrophages, comprising co-culturing microphages and apoptotic cells.

11. A preparation method of a composition for inhibiting cancer migration and invasion, comprising co-culturing microphages and apoptotic cells.

12. A method for inhibiting EMT (epithelial-mesenchymal transition) of cancer cells, comprising treating conditioned medium (CM) from macrophages exposed to apoptotic cancer cells to cancer cells.

13. A health functional food comprising conditioned medium (CM) from macrophages exposed to apoptotic cancer cells as an active ingredient for inhibiting cancer migration or invasion.

14. An anticancer adjuvant comprising conditioned medium (CM) from macrophages exposed to apoptotic cancer cells as an active ingredient for inhibiting EMT (epithelial-mesenchymal transition), migration and invasion of cancer cells.

15. A method for preventing or treating cancer comprising a step of administering conditioned medium (CM) from macrophages exposed to apoptotic cancer cells or apoptotic cancer cells to a subject with cancer.

16. A method for inhibiting EMT (epithelial-mesenchymal transition), migration or invasion of cancer cells comprising a step of administering conditioned medium (CM) from macrophages exposed to apoptotic cancer cells or apoptotic cancer cells to a subject with cancer.

17. A method for improving cancer treatment effect comprising a step of administering conditioned medium (CM) from macrophages exposed to apoptotic cancer cells or apoptotic cancer cells to a subject with cancer.

18. A use of conditioned medium (CM) from macrophages exposed to apoptotic cancer cells or apoptotic cancer cells for the prevention or treatment of cancer.

19. A use of conditioned medium (CM) from macrophages exposed to apoptotic cancer cells for inhibiting EMT (epithelial-mesenchymal transition), migration or invasion of cancer cells.

20. A use of conditioned medium (CM) from macrophages exposed to apoptotic cancer cells for the preparation of a health functional food for inhibiting migration or invasion of cancer cells.

21. A use of conditioned medium (CM) from macrophages exposed to apoptotic cancer cells for the preparation of an anticancer adjuvant for inhibiting EMT (epithelial-mesenchymal transition), migration or invasion of cancer cells.


<110>     Ewha University - Industry Collaboration Foundation

<120>     The composition for inhibiting cancer metastasis using apoptotic
          cells

<130>     2017FPO-01-012_PCT

<150>     KR 10-2016-0012493
<151>     2016-02-01

<150>     KR 10-2016-0162871
<151>     2016-12-01

<160>     46

<170>     KopatentIn 2.0

<210>     1
<211>     22
<212>     DNA
<213>     Artificial Sequence

<220>
<223>     Zeb1 forward primer


<400>     1
attcagctac tgtgagccct gc                                                22


<210>     2
<211>     22
<212>     DNA
<213>     Artificial Sequence

<220>
<223>     Zeb1 reverse primer


<400>     2
cattctggtc ctccacagtg ga                                                22


<210>     3
<211>     23
<212>     DNA
<213>     Artificial Sequence

<220>
<223>     Cdh2 forward primer


<400>     3
cctccagagt ttactgccat gac                                               23


<210>     4
<211>     22
<212>     DNA
<213>     Artificial Sequence

<220>
<223>     Cdh2 reverse primer

<400>    4
ccaccactga ttctgtatgc cg                                                22

<210>    5
<211>    20
<212>    DNA
<213>    Artificial Sequence

<220>
<223>    Vim forward primer

<400>    5
gcgtgcggct gcttcaagac                                                   20

<210>    6
<211>    20
<212>    DNA
<213>    Artificial Sequence

<220>
<223>    Vim reverse primer

<400>    6
atggcgtcgg ccagcgagaa                                                   20

<210>    7
<211>    20
<212>    DNA
<213>    Artificial Sequence

<220>
<223>    Cdh1 forward primer

<400>    7
tacggcggtg gtgaggacga                                                   20

<210>    8
<211>    20
<212>    DNA
<213>    Artificial Sequence

<220>
<223>    Cdh1 reverse primer

<400>    8
gccacacggg ggagacttgc                                                   20

<210>    9
<211>    21
<212>    DNA
<213>    Artificial Sequence

<220>
<223>    Crb3 forward primer


<400>    9
cggacccttt cacaaatagc a                                                21


<210>    10
<211>    20
<212>    DNA
<213>    Artificial Sequence

<220>
<223>    Crb3 reverse primer


<400>    10
cgttggactc atcacctggg                                                  20


<210>    11
<211>    20
<212>    DNA
<213>    Artificial Sequence

<220>
<223>    Cldn3 forward primer


<400>    11
cacctgacta ccgggcctag                                                  20


<210>    12
<211>    21
<212>    DNA
<213>    Artificial Sequence

<220>
<223>    Cldn3 reverse primer


<400>    12
ggtttctttg tccattcggc t                                                21


<210>    13
<211>    22
<212>    DNA
<213>    Artificial Sequence

<220>
<223>    Par6b forward primer


<400>    13
tttccaccgc caatccactg ct                                               22


<210>    14
<211>    22
<212>    DNA

<213>    Artificial Sequence

<220>
<223>    Par6b reverse primer


<400>    14
gctgatgacg atgtgaggct tc                                                22


<210>    15
<211>    20
<212>    DNA
<213>    Artificial Sequence

<220>
<223>    CD90 forward primer


<400>    15
gtcaggctgg tcaccttctg                                                   20


<210>    16
<211>    20
<212>    DNA
<213>    Artificial Sequence

<220>
<223>    CD90 reverse primer


<400>    16
aactcttggc accatgaacc                                                   20


<210>    17
<211>    20
<212>    DNA
<213>    Artificial Sequence

<220>
<223>    Oct4 forward primer


<400>    17
tcttctgctt cagcagcttg                                                   20


<210>    18
<211>    20
<212>    DNA
<213>    Artificial Sequence

<220>
<223>    Oct4 reverse primer


<400>    18
gttggagaag gtggaaccaa                                                   20


<210>    19

<211>    20
<212>    DNA
<213>    Artificial Sequence

<220>
<223>    CD44 forward primer


<400>    19
agaaatgggt tcagtgtggc                                                    20


<210>    20
<211>    20
<212>    DNA
<213>    Artificial Sequence

<220>
<223>    CD44 reverse primer


<400>    20
ttgctaccac atcaagctgc                                                    20


<210>    21
<211>    20
<212>    DNA
<213>    Artificial Sequence

<220>
<223>    CD133 forward primer


<400>    21
tagagggaag tcattcggct                                                    20


<210>    22
<211>    21
<212>    DNA
<213>    Artificial Sequence

<220>
<223>    CD133 reverse primer


<400>    22
cccaagatac cttcaatgct g                                                  21


<210>    23
<211>    22
<212>    DNA
<213>    Artificial Sequence

<220>
<223>    Snai1 forward primer


<400>    23
tgccctcaag atgcacatcc ga                                                 22

<210>    24
<211>    21
<212>    DNA
<213>    Artificial Sequence

<220>
<223>    Snail reverse primer


<400>    24
gggacaggag aagggcttct c                                                21


<210>    25
<211>    23
<212>    DNA
<213>    Artificial Sequence

<220>
<223>    Cdh2 forward primer


<400>    25
cctccagagt ttactgccat gac                                              23


<210>    26
<211>    23
<212>    DNA
<213>    Artificial Sequence

<220>
<223>    Cdh2 reverse primer


<400>    26
cctccagagt ttactgccat gac                                              23


<210>    27
<211>    22
<212>    DNA
<213>    Artificial Sequence

<220>
<223>    Vim forward primer


<400>    27
aggcaaagca ggagtccact ga                                               22


<210>    28
<211>    22
<212>    DNA
<213>    Artificial Sequence

<220>
<223>    Vim reverse primer


<400>    28

atctggcgtt ccagggactc at 22

<210> 29
<211> 23
<212> DNA
<213> Artificial Sequence

<220>
<223> Cdh1 forward primer

<400> 29
gcctcctgaa aagagagtgg aag 23

<210> 30
<211> 22
<212> DNA
<213> Artificial Sequence

<220>
<223> Cdh1 reverse primer

<400> 30
tggcagtgtc tctccaaatc cg 22

<210> 31
<211> 22
<212> DNA
<213> Artificial Sequence

<220>
<223> Par6b forward primer

<400> 31
gtttcaacgg ccaatccact gc 22

<210> 32
<211> 23
<212> DNA
<213> Artificial Sequence

<220>
<223> Par6b reverse primer

<400> 32
ctatggttgt caggacgcaa tac 23

<210> 33
<211> 23
<212> DNA
<213> Artificial Sequence

<220>
<223> OCLN forward primer

<400>    33
atggcaaagt gaatgacaag cgg                                              23


<210>    34
<211>    22
<212>    DNA
<213>    Artificial Sequence

<220>
<223>    OCLN reverse primer


<400>    34
ctgtaacgag gctgcctgaa gt                                               22


<210>    35
<211>    20
<212>    DNA
<213>    Artificial Sequence

<220>
<223>    ALDH1A forward primer


<400>    35
ccactcactg aatcatgcca                                                  20


<210>    36
<211>    20
<212>    DNA
<213>    Artificial Sequence

<220>
<223>    ALDH1A reverse primer


<400>    36
gcacgccaga cttacctgtc                                                  20


<210>    37
<211>    20
<212>    DNA
<213>    Artificial Sequence

<220>
<223>    CD44 forward primer


<400>    37
cacgtggaat acacctgcaa                                                  20


<210>    38
<211>    19
<212>    DNA
<213>    Artificial Sequence

<220>

<223>    CD44 reverse primer

<400>    38
gacaagtttt ggtggcacg                                                    19

<210>    39
<211>    20
<212>    DNA
<213>    Artificial Sequence

<220>
<223>    CD90 forward primer

<400>    39
gggagacctg caagactgtt                                                   20

<210>    40
<211>    17
<212>    DNA
<213>    Artificial Sequence

<220>
<223>    CD90 reverse primer

<400>    40
cggaagaccc cagtcca                                                      17

<210>    41
<211>    19
<212>    RNA
<213>    Artificial Sequence

<220>
<223>    PPAR gamma sense

<400>    41
aguauggugu ccaugagau                                                    19

<210>    42
<211>    19
<212>    RNA
<213>    Artificial Sequence

<220>
<223>    PPAR gamma antisense

<400>    42
aucucaugga caccauacu                                                    19

<210>    43
<211>    19
<212>    RNA
<213>    Artificial Sequence

<220>
<223>    PTEN sense

<400>    43
caggaaugaa ccaucuaca                                                    19

<210>    44
<211>    19
<212>    RNA
<213>    Artificial Sequence

<220>
<223>    PTEN antisense

<400>    44
uguagauggu ucauuccug                                                    19

<210>    45
<211>    25
<212>    RNA
<213>    Artificial Sequence

<220>
<223>    L-PGDS sense

<400>    45
caacuaugac gaguacgcuc ugcua                                             25

<210>    46
<211>    25
<212>    RNA
<213>    Artificial Sequence

<220>
<223>    L-PGDS antisense

<400>    46
gacuuccgca uggccacccu cuaca                                             25